# EUROPEAN PATENT APPLICATION

(11) **EP 4 566 619 A1**
(43) Date of publication of application: **11.06.2025**
(21) Application number: 23215236.3
(22) Date of filing: 08.12.2023
(51) Int. Cl.: A61K 39/00, C12N 15/11, C12N 15/113

(54) **TARGETING MODULES AGAINST CD19 AND CD20 FOR USE IN A METHOD FOR STIMULATING A REVERSED CHIMERIC ANTIGEN RECEPTOR-MEDIATED IMMUNE RESPONSE IN A MAMMAL**

(71) Applicant: AvenCell Therapeutics Inc., Watertown, MA 02472 (US)
(72) Inventor: MEYER, Jan-Erik, 01307 Dresden (DE); SPEHR, Johannes, 01307 Dresden (DE); EHNINGER, Armin, 01307 Dresden (DE)
(74) Representative: Kailuweit & Uhlemann Patentanwälte Partnerschaft mbB

(57) **Abstract**

The present invention relates to a targeting module comprising at least one CD19-binding domain, at least one CD20-binding domain and a tag or tag-binding domain, a pharmaceutical composition and a kit comprising the targeting module and a vector or a cell comprising a nucleotide sequence encoding a reversible chimeric antigen receptor.

## Description

The present invention relates to a targeting module comprising at least one CD19-binding domain, at least one CD20-binding domain and a tag or tag-binding domain, a pharmaceutical composition and a kit comprising the targeting module and a vector or a cell comprising a nucleotide sequence encoding a reversible chimeric antigen receptor.

Chimeric antigen receptors (CARs) are artificial receptors consisting of a binding moiety, which provides the antigen-specificity, e.g. a single chain variable fragment (scFv) recognizing a surface antigen of a tumor cell, a transmembrane domain, and one or several signaling chains derived from immune receptors, e.g. CD3ζ, that activates an immune cell (Cartellieri et al. 2010).

The first-generation CAR comprising one signaling chain was modified by adding a signal transduction domain of e.g. CD28 or CD137 (4-1BB), which are co-stimulatory molecules of a T cell, to increase the activation of immune cells (second generation CAR). A third generation CAR was developed by tandemly linking an additional complementary signal transduction domain derived from e.g. CD28, CD137 (4-1 BB) or CD134 (OX40), both which are tumor necrosis factor (TNF) receptor superfamily members, to a second-generation CAR. However, not all signal transduction domains derived from every T cell signal transducing protein sufficiently stimulate a T cell to damage and/or kill a target tumor cell. Therefore, finding intracellular signaling domains of signal transducing proteins that are effective when linked to a CAR is desirable.

Immune cells, genetically modified to express CARs, can be used to bind cells or tissue structures expressing the appropriate target of the CAR binding moiety. Cross-linking leads to an induction of signal pathways via the CAR signaling chains, which will change the biologic properties of the CAR-engrafted immune cell. In contrast, CAR activation in gene-modified regulatory T cells (Tregs) leads to an activation of Treg-specific immunomodulatory and suppressive mechanisms like interleukin (IL)-10 or tumor growth factor-beta (TGF-β) secretion. The adoptive transfer of immune cells engineered with chimeric antigen receptors (CARs) is currently considered a highly promising therapeutic option for the treatment of otherwise incurable malignant, infectious or autoimmune diseases.

However, the conventional CAR technology comes along with a number of critical issues, which need to be solved before this treatment modality can be widely applied for clinical treatments. First of all, several safety issues have to be addressed. So far, immune responses of T cells engineered with conventional CARs are difficult to control after infusion into the patient. Serious adverse event rates are high (Titov et al. 2018). Especially unexpected target gene expression on normal tissue may provoke a rapid and rigorous immune reaction of engineered T cells against normal cells, which can cause severe side effects (Morgan et al. 2010). Moreover, as CAR T cells are a new class of self-amplifying cell drugs, infused T cells can undergo a vigorous expansion in the presence of heavy tumor burden leading to tumor lysis syndrome, cytokine release syndrome and macrophage activation syndrome (Brudno and Kochenderfer 2016). Another drawback of conventional CAR technology is the restriction of engineered T cell retargeting to a single antigen. Such a monotherapeutic approach implies the risk for the development of tumor escape variants, which have lost the target antigen during treatment. The emergence of tumor escape variants under conventional CAR T cell therapy after several months was already observed in clinical trials (Sotillo et al. 2015). Taken together, these obstacles restrict the application of CAR T cells to very few indications. In fact, examples of clinical effectiveness have mostly been seen with CD19- and BCMA-targeting CAR T cells until now.

Modular switchable "universal" CAR T (UniCAR) approaches can overcome these limitations by separating antigen recognition and activating domain of a CAR into two separate operational units. T cells are engineered to express a CAR with a universal binding domain recognizing a tag (Cartellieri et al. 2016). Antigen-specificity is provided by soluble adapter molecules, which consist of an antigen-binding domain fused to the tag recognized by the UniCAR. Cartellieri et al. describe the treatment of CD33- and/or CD123-positive acute myeloid leukemia cells *in vitro* and *in vivo.*

Next to the UniCAR approach for recognizing various antigens (EP 2 990 416 A1) a reversed universal CAR (RevCAR) approach is known that promotes binding of an immune cell engineered to express a RevCAR comprising a tag to a target cell through an adaptor molecule comprising a tag-binding domain and a target cell binding domain (EP 3 581 200 A1).

Moreover, switchable CAR T approaches like UniCAR or RevCAR provide the possibility to rest CAR T cells in-between cycles of activation and stimulation by pausing administration of the soluble targeting module molecule. This is expected to prevent the exhaustion observed upon continuous stimulation of conventional CAR T cells thereby improving persistence (Weber et al. 2021).

Kittel-Boselli et al. describe targeting acute myeloid leukemia, in particular patient-derived AML cells expressing CD33 and CD123, using the RevCAR platform, wherein the RevCARs consist of the extracellular peptide epitope E5B9 or E7B6 and CD28 (28) hinge domain (HiD), CD28 transmembrane domain (TMD), the intracellular CD28 costimulatory (CSD) and CDS zeta (3z) activating signaling domain (ASD) (Kittel-Boselli et al. 2021). The targeting modules are constructed with the variable heavy (V_{H}) and light chain (V_{L}) domains derived from the monoclonal antibodies (mAbs) CD33, CD123, 5B9, or 7B6 connected via glycine (G)-serine (S) linkers.

WO 2016/154621 A1 describes switchable chimeric receptors comprising a non-antibody extracellular domain that interacts with a chimeric receptor binding partner presented on a switch. Furthermore, the chimeric receptor comprises a transmembrane domain and an intracellular signaling domain. The switch comprises the chimeric receptor binding partner, preferably an antibody or antibody fragment, and a targeting moiety. Preferably the chimeric receptor binding peptide comprises a peptide epitope tag. Preferably the chimeric receptor binding partner and the non-antibody extracellular domain are selected from the group consisting of isopeptag and pilin, spytag and spycatcher, SNARE and SNAP25, synaptobrevin and syntaxin, Hu-tag and RNAse I, K4 peptide and E4 peptide or a modified K4 peptide and a modified E4 peptide, a first mouse coronin 1A alpha helix and a second mouse coronin 1A alpha helix, an anchor domain of an A-kinase anchor protein and a regulatory subunit of cAMP-dependent protein kinase A (AD) and a dimerization and docking domain of cAMP-dependent protein kinase (DDD), barnase and barstar, coronin and ccCor1 peptide. The targeting moiety may target an antigen selected from CD19, Her2, CLL-1, CD33, EGFRvlll, CD20, CD22, BCMA or a fragment thereof.

Darowski et al. describe flexible chimeric antigen receptor adaptor molecules (CAR-adaptors) for the recruitment of chimeric antigen receptor T cells with tags like 5B9, GCN4, FITC, leucine zipper sequences, or biotinylated IgG, and targets like CD33, CD123, CD19, CD20, CD22, HER2, EGFR, CCR4, G2D, MCSP, ErbB2 (Darowski et al. 2019).

Feldmann et al. disclose RevCAR T cells efficiently kill tumor cells, flexibly redirected against multiple targets by exchanging the targeting modules, in particular targeting modules against PSMA and PSCA (Feldmann et al. 2020).

The object of the present invention is to provide an alternative targeting module for use in a switchable CAR system and/or a targeting module with prolonged half-life.

The object has been solved by a targeting module comprising at least one CD19-binding domain, at least one CD20-binding domain and a tag-binding domain or a tag, a pharmaceutical composition and a kit according to the independent claims. Advantageous embodiments of the invention are indicated in the dependent claims.

A first aspect of the invention provides a targeting module comprising
i) at least one CD19-binding domain comprising one of the sequences selected from SEQ ID No. 1 to SEQ ID No. 3 or a sequence identity of at least 95 % with one of the sequences selected from SEQ ID No. 1 to SEQ ID No. 3,
ii) at least one CD20-binding domain comprising SEQ ID No. 4, and
iii) a tag-binding domain or a tag.

Advantageously, the trispecific targeting module according to the invention targets both CD19 and CD20 simultaneously. Advantageously, the targeting module according to the invention has prolonged half-life. Advantageously, the targeting module according to the invention administered with a switchable chimeric antigen receptor could delay tumor growth, wherein the anti-tumor response of the switchable chimeric antigen receptor is only induced in the presence of the targeting module. The effect can be interrupted by withholding the administration of the targeting module to rest the switchable CAR-T cells and prevent their exhaustion. The switchable CAR-T cells can be reactivated and reexpanded by additional targeting module doses.

The targeting module according to the invention used in combination with a switchable chimeric antigen receptor is safer and more versatile than a classical CAR construct directed against a target. The versatility of the switchable CAR platform embodies a significant advantage for treating tumors with a highly heterogeneous antigenic profile. This therapy can be quickly adapted to the evolving antigenic profile to avoid a treatment-induced selection of tumor cells lacking the targeted antigen and the consequent tumor relapse. Moreover, only one switchable CAR-T cell product is required for different indications and targets.

As used herein, the term "targeting module" refers to a molecule, preferably a polypeptide or protein with at least three different domains, wherein each domain is specific for a target or a uniform group of targets, respectively, wherein at least two domains are specific for a target cell, in particular the CD19-binding domain and CD20-binding domain; and one domain is specific for a switchable chimeric antigen receptor, in particular the tag-binding domain or tag.

As used herein, the term "domain" refers to a part of a protein sequence, which can exist and function independently from the rest of the protein.

As used herein, the term "specific" refers to the ability of an antibody or antibody fragment or a protein, peptide or low molecular weight organic ligand to recognize and bind with a binding partner (e.g. a tumor antigen) protein present in a sample, but not substantially recognize or bind other molecules in the sample.

As used herein, the term "binds" or "binding" refers to a non-covalent binding, in particular ionic bonds, hydrogen bonds, Van der Waals forces and/or hydrophobic interactions.

In embodiments, the targeting module is isolated. In embodiments, the targeting module according to the invention is expressed as a recombinant protein. In further embodiments, the targeting module is chemically synthesized.

In embodiments, the targeting module is in monomeric, dimeric or polymeric form, preferably in monomeric form.

According to the invention, the targeting module comprises at least one CD19-binding domain. As used herein "CD19" (Cluster of Differentiation19) refers to a surface antigen expressed by B lymphocytes.

According to the invention, the targeting module comprises at least one CD19-binding domain comprising one of the sequences selected from SEQ ID No. 1 to SEQ ID No. 3 or a sequence identity of at least 95 % with one of the sequences selected from SEQ ID No. 1 to SEQ ID No. 3. As used herein, the term "% sequence identity" refers to the number of identical amino acid residues in relation to the length of the amino acid sequence. In embodiments, the CD19-binding domain comprises an amino acid sequence with at least 99% sequence identity with one of the sequences selected from SEQ ID No. 1 to SEQ ID No. 3.

In embodiments, the CD19-binding domain comprises an amino acid sequence with at least 95% sequence homology with one of the sequences selected from SEQ ID No. 1 to SEQ ID No. 3, preferably at least 99% sequence homology with one of the sequences selected from SEQ ID No. 1 to SEQ ID No. 3. As used herein, the term "% sequence homology" refers to the number of homologue amino acid residues in relation to the length of the amino acid sequence, wherein homologue amino acids are identical amino acids or amino acids comprising modifications selected from the group comprising D amino acids, pseudo peptide bonds, aminoalcohols, amino acids with modified side chains, in particular cysteine and selenocystein, and/or circular proteins. Advantageously, these homologues reveal increased stability.

In embodiments, the CD19-binding domain comprises an amino acid sequence with at least 95% sequence similarity with one of the sequences selected from SEQ ID No. 1 to SEQ ID No. 3, preferably at least 99% sequence similarity with one of the sequences selected from SEQ ID No. 1 to SEQ ID No. 3. As used herein, the term "similarity" refers to the sequence homology, wherein conservative substitutions of amino acid residues having similar physicochemical properties over the length of the amino acid sequence are comprised. The % sequence similarity is determined with any reasonable similarity-scoring matrix known by the person skilled in the art, preferably with a similarity-scoring matrix selected from BLOSUM50, BLOSUM62, PMBEC or VTML10 to VTM L80.

In embodiments, the at least one CD19-binding domain comprises CDR sequences according SEQ ID No. 8 to SEQ ID No. 13. As used herein, the term "CDR (Complementarity-determining regions)" refers to parts of the variable chains in antibodies or antibody fragments, where the antibodies or antibody fragments bind to their specific antigen. An antibody comprises three CDRs (CDR1, CDR2 and CDR3), arranged non-consecutively, on the amino acid sequence of each variable domain and thus, six CDRs on the two variable domains (V_{H} and V_{L}), which can come into contact with the antigen. A single-domain antibody (sdAb) comprises three CDRs (CDR1, CDR2 and CDR3), arranged non-consecutively, on the amino acid sequence of its variable domain, which can come into contact with the antigen.

According to the invention, the targeting module comprises at least one CD20-binding domain. As used herein "CD20" (Cluster of Differentiation20) refers to a glycosylated phosphoprotein expressed on the membrane surface of B cells.

According to the invention, the targeting module comprises at least one CD20-binding domain comprising SEQ ID No. 4, wherein X₅, X₁₄, X₄₃, X₄₄, X₄₅, X₆₂, X₆₅, X₇₅, X₇₆, X₇₉, X₈₇, X₈₈ and X₁₁₆ are independently from each other selected from a proteinogenic alpha-amino acid residue.

In some embodiments, X₅ to X₁₁₆ are selected as follows:
X₅ is selected from Glutamine or Valine;
X₁₄ is selected from hydrophobic residues, such as Isoleucine, Leucine, Valine, Alanine, Methionine, Phenylalanine, Proline and Tryptophan; preferably Alanine or Proline;
X₄₃ is selected from Lysine or Glutamine;
X₄₄ is selected from Glutamate or Glycine;
X₄₅ is selected from Arginine or Leucine;
X₆₂ is selected from Alanine or Aspartate;
X₆₅ is selected from polar and basic residues, such as Lysine, Arginine and Histidine, preferably Arginine or Lysine;
X₇₅ is selected from Alanine or Serine;
X₇₆ is selected from Glutamate or Lysine;
X₇₉ is selected from hydrophobic residues, such as Isoleucine, Leucine, Valine, Alanine, Methionine, Phenylalanine, Proline and Tryptophan; preferably Valine or Leucine;
X₈₇ is selected from polar and basic residues, such as Lysine, Arginine and Histidine, preferably Arginine or Lysine;
X₈₈ is selected from hydrophobic residues, such as Isoleucine, Leucine, Valine, Alanine, Methionine, Phenylalanine, Proline and Tryptophan; preferably Alanine or Proline; and
X₁₁₆ is selected from Glutamine or Leucine.

In embodiments, the targeting module comprises at least one CD20-binding domain comprising one of the sequences selected from SEQ ID No. 5 to SEQ ID No. 7 or with a sequence identity of at least 95% with one of the sequences selected from SEQ ID No. 5 to SEQ ID No. 7. In embodiments, the CD20-binding domain comprises an amino acid sequence with at least 99% sequence identity with one of the sequences selected from SEQ ID No. 5 to SEQ ID No. 7.

In embodiments, the CD20-binding domain comprises an amino acid sequence with at least 95% sequence homology with one of the sequences selected from SEQ ID No. 5 to SEQ ID No. 7, preferably at least 99% sequence homology with one of the sequences selected from SEQ ID No. 5 to SEQ ID No. 7.

In embodiments, the CD20-binding domain comprises an amino acid sequence with at least 95% sequence similarity with one of the sequences selected from SEQ ID No. 5 to SEQ ID No. 7, preferably at least 99% sequence similarity with one of the sequences selected from SEQ ID No. 5 to SEQ ID No. 7.

In embodiments, the at least one CD20-binding domain comprises CDR sequences according SEQ ID No. 14 to SEQ ID No. 16 or SEQ ID No. 14, SEQ ID No. 16 and SEQ ID No. 17.

In embodiments, the CD19-binding domain and/or the CD20-binding domain independently selected from antibody and antigen-binding fragment.

As used herein, the term "antibody" refers to a protein, which binds antigens via the antigen-binding fragment variable region (Fab). This is composed of one constant and one variable domain of each of the heavy (V_{H}) and the light chain (V_{L}). As used herein, the term "antibody fragment" or "antigen-binding fragment" refers to a protein comprising at least the V_{L} or V_{H} of an antibody. In embodiments, antibody fragments are selected from single-chain variable fragments (scFv), single-chain antibodies, Fc fragment, F(ab')2 fragments, Fab fragments, and fragments produced by a Fab expression library or single-domain antibodies (nanobodies).

As used herein, the term "single-chain variable fragment (scFv)" refers to an artificial antibody fragment comprising a variable domain of a light chain and a variable domain of a heavy chain of an antibody covalently linked. In embodiments, the V_{L} and V_{H} of an antibody are covalently linked by a short peptide of 10 to 25 amino acids. In further embodiments, the short peptide links the N-terminus of the V_{H} with the C-terminus of the V_{L}, or vice versa.

As used herein, the term "Fab fragments" refers to an antibody fragment comprisingone constant and one variable domain of each of the heavy and the light chain.

As used herein, the term "single-domain antibody (sdAb)" or "nanobody" is an antibody fragment consisting of a single monomeric variable antibody domain, in particular a VHH fragment.

In embodiments, the antibody is obtained from an animal species, preferably from a mammal such as human, simian, mouse, rat, rabbit, lama, alpaca, camel, shark, guinea pig, horse, cow, sheep, goat, pig, dog or cat. Preferably, the antibody or antibody fragment is a human, humanized or deimmunized antibody. Humanized antibodies can be prepared in various ways, for example, by resurfacing and CDR grafting. In case of resurfacing, a combination of molecular modeling, statistical analyses, and mutagenesis is used to modify all non-CDR regions on the surface of the antibody to become similar to the surface of antibodies of the target organism. In CDR grafting, the CDR regions according to the invention are introduced into known human framework regions, which are similar in sequence to the original ones. Deimmunized antibodies can be obtained by specifically mutating residues that confer immunogenicity hotspots as predicted based on in silico peptide-MHC affinity prediction.

In embodiments, the antibody or antibody fragment is a polyclonal, a monoclonal or a chimeric antibody, wherein an antigen-binding region of a non-human antibody is transferred into the framework of a human antibody by recombinant DNA techniques including in silico design.

In embodiments, antibodies to a selected tag or antigen may be produced by immunization of various hosts including, but not limited to, goats, rabbits, rats, mice, humans, through injection with cells expressing a particular protein, DNA or RNA encoding for the protein, the protein itself or any portion, fragment or oligopeptide that retain immunogenic properties of the protein.

In embodiments, the CD19-binding domain and/or the CD20-binding domain are antigen-binding fragments.

In embodiments, the CD19-binding domain and/or the CD20-binding domain are independently selected from single-chain variable fragments (scFv), single-chain antibodies, Fc fragment, F(ab')2 fragments, Fab fragments, and fragments produced by a Fab expression library or single-domain antibodies (nanobodies).

In embodiments, both the CD19-binding domain and the CD20-binding domain are VHH fragments.

In embodiments, the CD19-binding domain is a VHH. In embodiments, the CD20-binding domain is an scFv fragment.

In embodiments, the CD19-binding domain is an scFv. In embodiments, the CD20-binding domain is a VHH fragment.

In embodiments, the variable region(s) of the at least one CD19-binding domain and/or at least one CD20-binding domain comprise(s) a humanized amino acid sequence.

In embodiments, the targeting module comprises
i) at least one CD19-binding domain comprising one of the sequences selected from SEQ ID No. 1 to SEQ ID No. 3 or a sequence identity of at least 95 % with one of the sequences selected from SEQ ID No. 1 to SEQ ID No. 3,
ii) at least one CD20-binding domain comprising SEQ ID No. 4, and
iii) a tag.

As used herein, the term "tag" refers to a marker, in particular a peptide sequence or an organic molecule, attached to peptides or proteins to enable them to bind to specific atoms, ions or molecules, in particular the tag-binding domain.

In embodiments, the tag is selected from organic molecules including fluorescence labels, e.g., FITC (Fluorescein isothiocyanate), and biotin.

In embodiments, the tag is a peptide epitope tag. In further embodiments, the tag comprises 10 to 20 amino acids.

In embodiments, the peptide epitope tag is a myc-tag, a His-tag, a peptide sequence from yeast transcription factor GCN4, preferably according to SEQ ID No. 18, SEQ ID No. 19 or mutants thereof; a leucine zipper sequence, preferably SYNZIP 1 to SYNZIP 48, BATF, FOS, ATF4, ATF3, BACH1, JUND, NFE2L3, HEPTAD (Reinke et al. 2010), a sequence according toSEQ ID No. 20 or SEQ ID No. 21 or mutants thereof; or a peptide sequence from a human nuclear protein. In embodiments, the peptide epitope tag is a myc-tag, a His-tag, a peptide sequence from yeast transcription factor GCN4 according to SEQ ID No. 18, SEQ ID No. 19, a leucine zipper sequence selected from SYNZIP 1 to SYNZIP 48, BATF, FOS, ATF4, ATF3, BACH1, JUND, NFE2L3, HEPTAD (Reinke et al. 2010), a sequence according to SEQ ID No. 20 or SEQ ID No. 21 or a peptide sequence from a human nuclear protein.

In embodiments, the tag is a peptide sequence from the human La protein, in particular according to SEQ ID No. 22, SEQ ID No. 23, SEQ ID No. 24 or mutants thereof.

In embodiments, the tag is the human La epitope E5B9 according to SEQ ID No. 22 or E7B6 according to SEQ ID No. 23 or SEQ ID No. 24, preferably the human La epitope E5B9 according to SEQ ID No. 23 or E7B6 according to SEQ ID No. 24.

As used herein, the term "nuclear protein" refers to a protein found in the cell nucleus. Advantageously, tags, which are peptide sequences from nuclear antigens, cannot be accessed and bound by the corresponding tag-binding domain in the context of the native protein under physiological conditions. Further advantageously, the tag is not immunogenic. This leads to minimization of the risk of uncontrolled on-target off-site toxicities by CAR-expressing immune cells like the release of toxic levels of cytokines, referred to variously as cytokine storms or cytokine release syndrome (CRS).

In embodiments, the His-tag is an amino acid sequence consisting of histidine residues, preferably in the range of six to fourteen histidine residues.

In embodiments, the targeting module comprises
i) at least one CD19-binding domain comprising one of the sequences selected from SEQ ID No. 1 to SEQ ID No. 3 or a sequence identity of at least 95 % with one of the sequences selected from SEQ ID No. 1 to SEQ ID No. 3,
ii) at least one CD20-binding domain comprising SEQ ID No. 4, and
iii) a tag-binding domain.

In embodiments, the tag-binding domain is an antibody or antigen-binding fragment.

In embodiments, the tag-binding domain is an antibody or an antigen-binding fragment binding to a myc-tag, a His-tag, a peptide sequence from yeast transcription factor GCN4, preferably according to SEQ ID No. 18, SEQ ID No. 19 or mutants thereof; a leucine zipper sequence, preferably SYNZIP 1 to SYNZIP 48, BATF, FOS, ATF4, ATF3, BACH1, JUND, NFE2L3, HEPTAD (Reinke et al. 2010), a sequence according to SEQ ID No. 20 or SEQ ID No. 21 or mutants thereof; or a peptide sequence from a human nuclear protein. In embodiments, the tag-binding domain is an antibody or an antigen-binding fragment binding to a myc-tag, a His-tag, a peptide sequence from yeast transcription factor GCN4 according to SEQ ID No. 18, SEQ ID No. 19, a leucine zipper sequence selected from SYNZIP 1 to SYNZIP 48, BATF, FOS, ATF4, ATF3, BACH1, JUND, NFE2L3, HEPTAD (Reinke et al. 2010), a sequence according toSEQ ID No. 20 or SEQ ID No. 21 or a peptide sequence from a human nuclear protein.

In embodiments, the tag-binding domain is an antigen-binding fragment. In embodiments, the tag-binding domain is an scFv or a Fab fragment.

In embodiments, the tag-binding domain is an scFv binding a La epitope. In embodiments, the tag-binding domain is an scFv binding La epitope 5B9 or 7B6 according to SEQ ID No. 22 or SEQ ID No. 24.

In embodiments, V_{L} and V_{H} are connected via a glycine-serine linker with the structure (GₓS_{y}) with x and y selected from 1 to 10, preferably 3 to 5. Mostly preferred are 1 to 10 repeats of the sequence G₄S₁ (SEQ ID No. 25). In embodiments, linkers are used that are constituted of a peptide sequence that can increase the protease resistance of the antibody derivatives. As used herein, the term "linker" (also spacer) refers to a molecule or molecule part separating at least two elements under consideration, in particular selected from functional groups, tags, binding domains or binding domain subunits, such as a V_{L} and a V_{H} domain.

In embodiments, the linker comprises 20 to 30 amino acids, preferably 25 amino acids.

In embodiments, the linker is an amino acid sequence according SEQ ID No. 26 or SEQ ID No. 27.

In embodiments, the tag-binding domain binding a human La epitope E5B9 comprises CDR sequences according to SEQ ID No. 28, SEQ ID No. 29, SEQ ID No. 30, SEQ ID No. 31, amino acid sequence WAS (Trp-Ala-Ser) and SEQ ID No. 32.

In embodiments, the tag-binding domain is an antibody or an antigen-binding fragment comprising a V_{L} according to the following sequence: DIVMTQSPDSLAVSLGERATINCX₂₄SSQSLLNSRTX₃₅KNYLAWYQQKPGQPPKLLIYWASTR X₆₁SGVPDRFSGSGSGTDFTLTISSLQAEDVAVYYCKQSYNLX₁₀₁TFGGGTKVEIK (SEQ ID No. 33), wherein X₂₄, X₃₅, X₆₁ and X₁₀₁ are independently from each other selected from a proteinogenic alpha-amino acid residue; or an amino acid sequence having at least 90 % sequence identity, preferably at least 95% sequence identity; to sequence SEQ ID No. 34 or SEQ ID No. 36.

In some embodiments, X₂₄ to X₁₀₁ are selected as follows:
X₂₄ is selected from polar and/or positive charged residues, such as Serine, Threonine, Asparagine, Glutamine, Histidine, Lysine and Arginine; preferably Lysine or Arginine;
X₃₅ is preferably selected from Lysine and Proline;
X₆₁ is selected from polar and charged residues, such as Asparagine, Aspartic Acid, Glutamine, Glutamic acid, Histidine, Lysine and Arginine, preferably Glutamic acid and Lysine;
X₁₀₁ is selected from hydrophobic residues, such as Isoleucine, Leucine, Valine, Alanine, Methionine, Phenylalanine, Proline and Tryptophan; preferably Leucine or Proline.

In embodiments, the tag-binding domain is an antibody or an antigen-binding fragment comprising a V_{H} with an amino acid sequence having at least 90 % sequence identity, preferably at least 95 % sequence identity; to sequence SEQ ID No. 35 or SEQ ID No. 37.

In embodiments, the tag-binding domain comprises a sequence having each at least 90 % sequence identity, preferably at least 95 % sequence identity; to the sequences according to SEQ ID No. 34 (V_{L}) and SEQ ID No. 35 (V_{H}).

In embodiments, the tag-binding domain is an anti-La 5B9 scFv according to SEQ ID No. 34 (V_{L}) and SEQ ID No. 35 (V_{H}).

In embodiments, the tag-binding domain comprises a sequence having each at least 90 % sequence identity, preferably at least 95 % sequence identity; to the sequences according to SEQ ID No. 36 (V_{L}) and SEQ ID No. 37 (V_{H}).

In embodiments, the tag-binding domain is an anti-La 7B6 scFv according to SEQ ID No. 36 (V_{L}) and SEQ ID No. 37 (V_{H}).

In embodiments, the tag-binding domain comprises a V_{L}-linker-V_{H} structure, wherein the V_{L} region of the tag-binding domain comprises a sequence with at least 95 % identity, preferably 99 % identity, with the sequence according to SEQ ID No. 34 and/or the V_{H} region of the tag-binding domain comprises a sequence with at least 95 % identity, preferably 99 % identity, with the sequence according to SEQ ID No. 35. As used herein, the term "V_{L}-linker-V_{H} structure" refers to a structure, wherein the C-terminus of the V_{L} region is connected with a linker, which is connected to the N-terminus of the V_{H} region.

In embodiments, the different domains of the targeting module are linked with each other by a linker. The linker comprises a short sequence of preferably 10 to 20 amino acid residues. In embodiments, the targeting module comprises a flexible peptide sequence that is selected such that the domains have a three-dimensional folding that allows them to exhibit the specificity for effector cell and target cell binding. Preferred linkers are glycine-serine linkers with the structure (GₓS_{y}) with x and y selected from 1 to 10, preferably 1 to 5. Mostly preferred are 1 to 10 repeats of the sequence G₄S₁ (SEQ ID No. 25). Moreover, linkers are preferred that are constituted of a peptide sequence that can increase the protease resistance of the antibody derivatives.

In embodiments, the linker is SEQ ID No. 26 or SEQ ID No. 27.

In embodiments, the tag-binding domain is a Fab fragment binding a La epitope. In embodiments, the tag-binding domain is a Fab fragment binding La epitope 5B9 comprising an amino acid sequence according to SEQ ID No. 38 and SEQ ID No. 39.

In embodiments, the targeting module comprises a further domain selected from the group comprising co-stimulatory ligands, radionuclides, cell death-inducing chemical compounds and half-life increasing domains, preferably IgG1 Fc, IgG2 Fc, IgG3 Fc, IgG4 Fc, HSA, FcRn-binding peptides or mutants thereof. As used herein, the term "mutants" refers to proteins having at least 90% sequence identity to the half-life increasing domain, preferably at least 95% sequence identity. Advantageously, the mutant is capable of having one or more activities of the named peptides or proteins; in particular, the mutant increases the half-life like the half-life increasing domain.

In embodiments, the tag-binding domain comprises at least one half-life increasing domain. In embodiments, the tag-binding domain comprises at least one half-life increasing domain selected from an amino acid sequence according to SEQ ID No. 40 or SEQ ID No. 41.

In embodiments, the tag-binding domain comprises at least one leader peptide. As used herein, the term "leader peptide" (also signal peptide) refers to a short amino acid sequence at the N-terminus of proteins meant for secretion or membrane localization. In embodiments, the tag-binding domain comprises at least one leader peptide selected from an amino acid sequence according to SEQ ID No. 42 or SEQ ID No. 43.

In embodiments, the length of the targeting module is in the range of 200 to 1600 amino acids, preferably 600 to 1600 amino acids.

In embodiments, the targeting module comprises three chains, wherein the length of the chains is independently from each other in the range of 200 to 550 amino acids.

In embodiments, the targeting module comprises at least three chains comprising sequences from the group comprising SEQ ID No. 44 to SEQ ID No. 76.

In embodiments, the targeting module comprises at least three chains comprising
- one amino acid sequence selected from the group comprising SEQ ID No. 39, SEQ ID No. 46, SEQ ID No. 47, SEQ ID No. 72, SEQ ID No. 73, SEQ ID No. 74, SEQ ID No. 75, and SEQ ID No. 76, and
- one amino acid sequence selected from the group comprising SEQ ID No. 44 and SEQ ID No. 45, and
- one amino acid sequence selected from the group comprising SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, and SEQ ID No. 71.

In embodiments, the targeting module comprises
- SEQ ID No. 46, SEQ ID No.44 and SEQ ID No. 48, or
- SEQ ID No. 47, SEQ ID No.45 and SEQ ID No. 49, or
- SEQ ID No. 46, SEQ ID No.44 and SEQ ID No. 50, or
- SEQ ID No. 47, SEQ ID No.45 and SEQ ID No. 51, or
- SEQ ID No. 46, SEQ ID No.44 and SEQ ID No. 52, or
- SEQ ID No. 47, SEQ ID No.45 and SEQ ID No. 53, or
- SEQ ID No. 46, SEQ ID No.44 and SEQ ID No. 54, or
- SEQ ID No. 47, SEQ ID No.45 and SEQ ID No. 55, or
- SEQ ID No. 46, SEQ ID No.44 and SEQ ID No. 56, or
- SEQ ID No. 47, SEQ ID No.45 and SEQ ID No. 57, or
- SEQ ID No. 46, SEQ ID No.44 and SEQ ID No. 58, or
- SEQ ID No. 47, SEQ ID No.45 and SEQ ID No. 59, or
- SEQ ID No. 39, SEQ ID No.44 and SEQ ID No. 60, or
- SEQ ID No. 76, SEQ ID No.45 and SEQ ID No. 61, or
- SEQ ID No. 39, SEQ ID No.44 and SEQ ID No. 62, or
- SEQ ID No. 76, SEQ ID No.45 and SEQ ID No. 63, or
- SEQ ID No. 39, SEQ ID No.44 and SEQ ID No. 64, or
- SEQ ID No. 76, SEQ ID No.45 and SEQ ID No. 65, or
- SEQ ID No. 39, SEQ ID No.44 and SEQ ID No. 66, or
- SEQ ID No. 76, SEQ ID No.45 and SEQ ID No. 67, or
- SEQ ID No. 39, SEQ ID No.44 and SEQ ID No. 68, or
- SEQ ID No. 76, SEQ ID No.45 and SEQ ID No. 69, or
- SEQ ID No. 39, SEQ ID No.44 and SEQ ID No. 70, or
- SEQ ID No. 76, SEQ ID No.45 and SEQ ID No. 71, or
- SEQ ID No. 72, SEQ ID No.44 and SEQ ID No. 54, or
- SEQ ID No. 73, SEQ ID No.45 and SEQ ID No. 55, or
- SEQ ID No. 74, SEQ ID No.44 and SEQ ID No. 54, or
- SEQ ID No. 75, SEQ ID No.45 and SEQ ID No. 55, or
- SEQ ID No. 72, SEQ ID No.44 and SEQ ID No. 56, or
- SEQ ID No. 73, SEQ ID No.45 and SEQ ID No. 57, or
- SEQ ID No. 74, SEQ ID No.44 and SEQ ID No. 56, or
- SEQ ID No. 75, SEQ ID No.45 and SEQ ID No. 57, or
- SEQ ID No. 72, SEQ ID No.44 and SEQ ID No. 58, or
- SEQ ID No. 73, SEQ ID No.45 and SEQ ID No. 59, or
- SEQ ID No. 74, SEQ ID No.44 and SEQ ID No. 58, or
- SEQ ID No. 75, SEQ ID No.45 and SEQ ID No. 59, or
- SEQ ID No. 72, SEQ ID No.44 and SEQ ID No. 48, or
- SEQ ID No. 73, SEQ ID No.45 and SEQ ID No. 49, or
- SEQ ID No. 74, SEQ ID No.44 and SEQ ID No. 48, or
- SEQ ID No. 75, SEQ ID No.45 and SEQ ID No. 49, or
- SEQ ID No. 72, SEQ ID No.44 and SEQ ID No. 50, or
- SEQ ID No. 73, SEQ ID No.45 and SEQ ID No. 51, or
- SEQ ID No. 74, SEQ ID No.44 and SEQ ID No. 50, or
- SEQ ID No. 75, SEQ ID No.45 and SEQ ID No. 51, or
- SEQ ID No. 72, SEQ ID No.44 and SEQ ID No. 52, or
- SEQ ID No. 73, SEQ ID No.45 and SEQ ID No. 53, or
- SEQ ID No. 74, SEQ ID No.44 and SEQ ID No. 52, or
- SEQ ID No. 75, SEQ ID No.45 and SEQ ID No. 53.

In a further aspect, the invention is a nucleic acid, a vector or a cell comprising a nucleotide sequence encoding a targeting module according to the invention.

In embodiments, the nucleic acid, vector or cell comprises at least three nucleic acid sequences encoding one of the sequences according to SEQ ID No. 44 to SEQ ID No. 76.

In embodiments, the nucleic acid, vector or cell comprises at least three nucleic acid sequences encoding
- one amino acid sequence selected from the group comprising SEQ ID No. 39, SEQ ID No. 46, SEQ ID No. 47, SEQ ID No. 72, SEQ ID No. 73, SEQ ID No. 74, SEQ ID No. 75, and SEQ ID No. 76, and
- one amino acid sequence selected from the group comprising SEQ ID No. 44 and SEQ ID No. 45, and
- one amino acid sequence selected from the group comprising SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, and SEQ ID No. 71.

In embodiments, the nucleic acid, vector or cell comprises at least three nucleic acid sequences encoding SEQ ID No. 46, SEQ ID No.44 and SEQ ID No. 48, or
- SEQ ID No. 47, SEQ ID No.45 and SEQ ID No. 49, or
- SEQ ID No. 46, SEQ ID No.44 and SEQ ID No. 50, or
- SEQ ID No. 47, SEQ ID No.45 and SEQ ID No. 51, or
- SEQ ID No. 46, SEQ ID No.44 and SEQ ID No. 52, or
- SEQ ID No. 47, SEQ ID No.45 and SEQ ID No. 53, or
- SEQ ID No. 46, SEQ ID No.44 and SEQ ID No. 54, or
- SEQ ID No. 47, SEQ ID No.45 and SEQ ID No. 55, or
- SEQ ID No. 46, SEQ ID No.44 and SEQ ID No. 56, or
- SEQ ID No. 47, SEQ ID No.45 and SEQ ID No. 57, or
- SEQ ID No. 46, SEQ ID No.44 and SEQ ID No. 58, or
- SEQ ID No. 47, SEQ ID No.45 and SEQ ID No. 59, or
- SEQ ID No. 39, SEQ ID No.44 and SEQ ID No. 60, or
- SEQ ID No. 76, SEQ ID No.45 and SEQ ID No. 61, or
- SEQ ID No. 39, SEQ ID No.44 and SEQ ID No. 62, or
- SEQ ID No. 76, SEQ ID No.45 and SEQ ID No. 63, or
- SEQ ID No. 39, SEQ ID No.44 and SEQ ID No. 64, or
- SEQ ID No. 76, SEQ ID No.45 and SEQ ID No. 65, or
- SEQ ID No. 39, SEQ ID No.44 and SEQ ID No. 66, or
- SEQ ID No. 76, SEQ ID No.45 and SEQ ID No. 67, or
- SEQ ID No. 39, SEQ ID No.44 and SEQ ID No. 68, or
- SEQ ID No. 76, SEQ ID No.45 and SEQ ID No. 69, or
- SEQ ID No. 39, SEQ ID No.44 and SEQ ID No. 70, or
- SEQ ID No. 76, SEQ ID No.45 and SEQ ID No. 71, or
- SEQ ID No. 72, SEQ ID No.44 and SEQ ID No. 54, or
- SEQ ID No. 73, SEQ ID No.45 and SEQ ID No. 55, or
- SEQ ID No. 74, SEQ ID No.44 and SEQ ID No. 54, or
- SEQ ID No. 75, SEQ ID No.45 and SEQ ID No. 55, or
- SEQ ID No. 72, SEQ ID No.44 and SEQ ID No. 56, or
- SEQ ID No. 73, SEQ ID No.45 and SEQ ID No. 57, or
- SEQ ID No. 74, SEQ ID No.44 and SEQ ID No. 56, or
- SEQ ID No. 75, SEQ ID No.45 and SEQ ID No. 57, or
- SEQ ID No. 72, SEQ ID No.44 and SEQ ID No. 58, or
- SEQ ID No. 73, SEQ ID No.45 and SEQ ID No. 59, or
- SEQ ID No. 74, SEQ ID No.44 and SEQ ID No. 58, or
- SEQ ID No. 75, SEQ ID No.45 and SEQ ID No. 59, or
- SEQ ID No. 72, SEQ ID No.44 and SEQ ID No. 48, or
- SEQ ID No. 73, SEQ ID No.45 and SEQ ID No. 49, or
- SEQ ID No. 74, SEQ ID No.44 and SEQ ID No. 48, or
- SEQ ID No. 75, SEQ ID No.45 and SEQ ID No. 49, or
- SEQ ID No. 72, SEQ ID No.44 and SEQ ID No. 50, or
- SEQ ID No. 73, SEQ ID No.45 and SEQ ID No. 51, or
- SEQ ID No. 74, SEQ ID No.44 and SEQ ID No. 50, or
- SEQ ID No. 75, SEQ ID No.45 and SEQ ID No. 51, or
- SEQ ID No. 72, SEQ ID No.44 and SEQ ID No. 52, or
- SEQ ID No. 73, SEQ ID No.45 and SEQ ID No. 53, or
- SEQ ID No. 74, SEQ ID No.44 and SEQ ID No. 52, or
- SEQ ID No. 75, SEQ ID No.45 and SEQ ID No. 53.

According to the invention, the nucleic acid, vector and/or cell are isolated.

In embodiments, the nucleic acid is a cDNA. As used herein, the term "cDNA" (complementary DNA) refers to double-stranded DNA synthesized from a single-stranded RNA, e.g. mRNA, in a reaction catalyzed by the enzyme reverse transcriptase. In embodiments, cDNA is of synthetic origin. In further embodiments, cDNA is derived from mRNA, therefore containing only exons but no introns, as opposed to genomic DNA.

The vector is preferably a plasmid, an artificial chromosome, linearized DNA or RNA, a virus particle or another vector that contains an expression cassette that is incorporated stably into the genome of a host cell or host organism.

In embodiments, the cell is selected from immune cells, preferably with cytolytic, phagocytic or immunosuppressive activity, such as T cells, Natural Killer (NK) cells and macrophages. In preferred embodiments, the cell is selected from T cells, including alpha/beta and gamma/delta T cells or subpopulations of T cells like stem-cell memory T cells or central memory T cells, cytotoxic T cells or NK cells.

Another aspect of the invention is the use of the targeting module in the treatment of cancer, infectious disease or autoimmune disease,
wherein the targeting module is administered in combination with a cell comprising a nucleotide sequence encoding a switchable chimeric antigen receptor, wherein the switchable chimeric antigen receptor comprises
   - a tag-binding domain or a tag,
   - an extracellular hinge and a transmembrane domain and
   - a signal transduction domain,
wherein the tag-binding domain of the targeting module binds to the tag of the switchable chimeric antigen receptor or the tag of the targeting module binds to the tag-binding domain of the switchable chimeric antigen receptor.

The term "autoimmune disorder" refers to an abnormal immune response of the body against substances and tissues normally present in the body (autoimmunity).

As used herein, the term "switchable chimeric antigen receptor" refers to an artificial chimeric fusion protein, in particular a receptor comprising a tag-binding domain or a tag, an extracellular hinge and a transmembrane domain and a signal transduction domain. The domains can be derived from different sources and therefore, the receptor is called chimeric. Advantageously, the receptor can bind with the tag-binding domain or tag to different targeting modules.

Advantageously, the cell comprising a nucleotide sequence encoding a switchable CAR expresses the switchable CAR, which has binding specificity for the tag or tag-binding domain of the targeting module, which in turn binds to CD19 and CD20 on a target cell.

As used herein, the term "administered in combination" refers to a treatment, wherein the targeting module is administered prior to, simultaneously with and/or after the administration of the cell comprising a nucleotide sequence encoding a switchable chimeric antigen receptor (CAR).

In embodiments, the targeting module is administered on its own, preferably one hour to 2 days, more preferably 4 to 24 hours, prior to the administration of the cell comprising a nucleotide sequence encoding a switchable CAR. Advantageously, the administration of the targeting module prior to the administration of the cell comprising a nucleotide sequence encoding a switchable CAR stimulates the switchable CAR and increases the expansion of the switchable CAR carrying effector cells and their accumulation at the target site.

In embodiments, the targeting module is administered simultaneously with the cell comprising a nucleotide sequence encoding a switchable CAR.

In embodiments, the targeting module is administered until, preferably in the range of 3days to 30 days, after the administration of the cell comprising a nucleotide sequence encoding a switchable CAR. In embodiments, additional such doses of the targeting module may be administered following resting periods to reactivate the switchable CAR-carrying effector cells.

In embodiments, the targeting module is used in the treatment of cancer. In embodiments, the targeting module is used in the treatment of tumors of the hematopoietic and lymphoid tissues. In embodiments, the targeting module is used in the treatment of blood cancer. In embodiments, the targeting module is used in the treatment of b cell lymphomas.

In embodiments, the targeting module is administered in combination with a cell comprising a nucleotide sequence encoding a switchable chimeric antigen receptor and at least one further targeting module,
wherein the at least one further targeting module comprises at least one target cell-binding domain and a tag-binding domain or a tag,
wherein the at least one target cell-binding domain is an antibody, antibody fragment, a protein, a peptide or a low molecular weight organic ligand that binds to a surface antigen selected from the group comprising CD2, CD3, CD4, CD5, CD7, CD8, CD10, CD15, CD22, CD23, CD25, CD30, CD33, CD38, CD44, CD44v6, CD52, CD66a, CD66b, CD66c, CD66d, CD66e, CD66f, CD90, CD99, CD123, CD133, CD135, CD150, CD181, CD182, CD184, CD223, CD229, CD269, CD273, CD274, CD276, CD279, CD319, CD366, CD371, cytokine receptors, CXCR4, c-Met, mesothelin, a member of the epidermal growth factor receptor family or a mutant thereof, a member of the tumor necrosis factor receptor superfamily, a claudin, an ephrin, an ephrin receptor, a fucosyl transferase, a prostate specific antigen, an embryonic antigen, a member of the vascular endothelia growth factor family, epithelial cell adhesion molecule, alpha-fetoprotein, a member of the intercellular adhesion molecule family, a C-type lectin, an integrin, a member of the mucin protein family, a follicle-stimulating hormone receptor, a high molecular weight-melanoma associated antigen, a folate binding protein, a folate receptor, a somatostatin receptor, a ligand of the NKG2D receptor, a member of the epithelia glycoprotein family, a diasialogangloside, a glypican, a G protein-coupled receptor, a human papillomavirus protein, cancer/testis antigen, fibroblast activation protein, a member of the carbonic anhydrase family, a member of the carbohydrate antigen family, a Notch ligand, melanoma-associated chondroitin sulfate proteoglycan, glycoprotein A33, guanylate cyclase 2C and tumor-specific glycan,
wherein the targeting module and the at least one further targeting module comprise different target cell-binding domains, and identical tag-binding domains or tags.

As used herein, the term "target cell-binding domain" refers to a peptide, protein, or low molecular weight organic ligand, which specifically binds a protein or protein complex (antigen) on the surface of a target cell, in particular a cancer cell, T cell, infected cell, pathogens or parasites.

As used herein, the terms "peptide", "polypeptide" and "protein" are used interchangeably and refer to a compound comprised of amino acid residues covalently linked by peptide bonds. A protein or peptide must contain at least two amino acids, and no limitation is placed on the maximum number of amino acids that can comprise a protein's or peptide's sequence.

As used herein, the term "low molecular weight organic ligand" refers to an organic molecule with a molecular weight of maximal 10 kilodaltons, preferably of maximal 3 kilodaltons, which specifically binds a protein or protein complex (antigen) on the surface of a target cell, preferably a cancer cell, T cell, infected cell or pathogens or parasites.

The term "target cell-binding domain" also comprises soluble T cell receptors, which are composed of the alpha and beta or the gamma and delta chains of a T cell receptor (TCR), fragments or mutants thereof. Such TCR-derived binding moieties recognize and bind to peptides presented by human leukocyte antigen class (HLA) I and II protein complexes. Examples are, but are not limited to, TCRs specific for peptides derived from proteins like EGFR family, survivin, srylike high motility group box (SOX) protein family, melanoma-associated antigens (e.g. auto immunogenic cancer/testis antigen NY-ESO-1, members of the melanoma antigen family A MAGEA, the preferentially expressed antigen in melanoma PRAME), and leukemia-associated antigens (e.g. Wilms tumor gene 1 WT1).

In embodiments, the target cell-binding domain is a soluble T cell receptor consisting of the alpha and beta or the gamma and delta chain of a T cell receptor (TCR).

In embodiments, the targeting module according to the invention is used in combination with the vector or cell comprising a nucleotide sequence encoding a switchable chimeric antigen receptor for the manufacture of a medicament for the treatment of cancer, infectious disease or autoimmune disease.

In embodiments, the targeting module according to the invention is used in a method for treatment cancer, infectious disease or autoimmune disease in a subject in need thereof,
wherein the targeting module is administered in combination with a cell comprising a nucleotide sequence encoding a switchable chimeric antigen receptor, wherein the switchable chimeric antigen receptor comprises
   - a tag-binding domain or a tag,
   - an extracellular hinge and a transmembrane domain and
   - a signal transduction domain,
wherein the tag-binding domain of the targeting module binds to the tag of the switchable chimeric antigen receptor or the tag of the targeting module binds to the tag-binding domain of the switchable chimeric antigen receptor.

Another aspect of the invention is a pharmaceutical composition comprising the targeting module and a pharmaceutically acceptable thinner or carrier.

In embodiments, the pharmaceutical composition is administered parenterally, particularly intravenously. In embodiments, the pharmaceutical composition is present in a form suitable for intravenous administration. In embodiments, the pharmaceutical composition is a solution, emulsion or suspension.

In embodiments, the pharmaceutical composition is an injectable buffered solution comprising a concentration in the range of 1 ng/ml to 500 mg/ml of the targeting module. In embodiments, the pharmaceutical composition comprises a concentration in the range of 50 µg/ml to 5 mg/ml of the targeting module.

The pharmaceutical composition comprises a pharmaceutically acceptable thinner (dilution agent) or carrier. In embodiments, the carrier is selected from water, an aqueous buffer solution, 0.9 % saline solution, 5 % glucose, 5 % xylitol, 0.3 % glycine solution, ringer solutions or amino acid solutions. In embodiments, the aqueous buffer solution is selected from an aqueous histidine, sodium succinate, sodium citrate, sodium acetate, sodium phosphate or potassium phosphatebuffered solution with a pH value in the range of pH 5.0 to pH 7.0. In embodiments, the aqueous buffer solution has a buffer concentration in the range of 1 mmol/l (mM) to 500 mM. In embodiments, the aqueous buffer solution has a buffer concentration in the range of 5 mM to 20 mM. In embodiments, the aqueous buffer solution has a buffer concentration in the range of 5 mM to 10 mM.

In embodiments, the carrier comprises sodium chloride. In embodiments, the carrier comprises sodium chloride with a concentration in the range of 1 mM to 300 mM. In embodiments, the carrier comprises sodium chloride with a concentration of about 150 mM.

In embodiments, the pharmaceutical composition comprises a stabilizer. In embodiments, the pharmaceutical composition comprises a stabilizer with a concentration in the range of 1 mM to 900 mM. In embodiments, the pharmaceutical composition comprises a stabilizer with a concentration in the range of 50 mM and 600 mM. In embodiments, the stabilizer is sucrose, trehalose or L-methionine.

In embodiments, the pharmaceutical composition comprises pharmaceutically acceptable excipients. The term "pharmaceutically acceptable excipients" refers to compounds, which provide approximately physiological conditions and/or increase the stability, such as agents for adjusting the pH value and buffering agents, agents for adjusting the toxicity and the like. In embodiments, pharmaceutically acceptable excipients are selected from sodium acetate, sodium chloride, potassium chloride, calcium chloride, sodium lactate and polysorbate-80, preferably polysorbate-80 in the range of 0.0001 % (w/v) to 1 % (w/v). In embodiments, the pharmaceutically acceptable excipient is in the range of 0.001 % (w/v) to 0.1 % (w/v).

In embodiments, the pharmaceutical composition comprises the targeting module in a dosage quantity in the range of 25 µg/day to 100 mg/day. In embodiments, the pharmaceutical composition comprises the targeting module in a dosage quantity in the range of 0.1 mg/day to 20 mg/day.

In embodiments, the pharmaceutical composition is sterile. In embodiments, the pharmaceutical composition sterilized by conventional well-known techniques including, but not limited to, sterile filtration.

In embodiments, the pharmaceutical composition is used for administration to a subject.

In embodiments, the pharmaceutical composition is lyophilized prior to storage or stored as solution at ambient temperature or below, including, but not limited to, frozen storage.

In embodiments, the pharmaceutical composition is reconstituted and/or diluted in an infusion and stabilizer solution prior to administration to a subject. The solutions used for reconstitution or infusion/stabilization may contain any of the components mentioned for the pharmaceutical composition or similar components.

In embodiments, the pharmaceutical composition comprises a cell comprising a nucleotide sequence encoding a switchable chimeric antigen receptor, wherein the switchable chimeric antigen receptor comprises
- a tag-binding domain or a tag,
- an extracellular hinge and a transmembrane domain and
- a signal transduction domain,
wherein the tag-binding domain of the targeting module binds to the tag of the switchable chimeric antigen receptor or the tag of the targeting module binds to the tag-binding domain of the switchable chimeric antigen receptor.

In embodiments, the pharmaceutical composition comprises at least one further targeting module, wherein the at least one further targeting module comprises at least one target cell-binding domain and a tag-binding domain or a tag,
wherein the at least one target cell-binding domain is an antibody, antibody fragment, a protein, a peptide or a low molecular weight organic ligand that binds to surface antigens selected from the group comprising CD2, CD3, CD4, CD5, CD7, CD8, CD10, CD15, CD22, CD23, CD25, CD30, CD33, CD38, CD44, CD44v6, CD52, CD66a, CD66b, CD66c, CD66d, CD66e, CD66f, CD90, CD99, CD123, CD133, CD135, CD150, CD181, CD182, CD184, CD223, CD229, CD269, CD273, CD274, CD276, CD279, CD319, CD366, CD371, cytokine receptors, CXCR4, c-Met, mesothelin, a member of the epidermal growth factor receptor family or a mutant thereof, a member of the tumor necrosis factor receptor superfamily, a claudin, an ephrin, an ephrin receptor, a fucosyl transferase, a prostate specific antigen, an embryonic antigen, a member of the vascular endothelia growth factor family, epithelial cell adhesion molecule, alpha-fetoprotein, a member of the intercellular adhesion molecule family, a C-type lectin, an integrin, a member of the mucin protein family, a follicle-stimulating hormone receptor, a high molecular weight-melanoma associated antigen, a folate binding protein, a folate receptor, a somatostatin receptor, a ligand of the NKG2D receptor, a member of the epithelia glycoprotein family, a diasialoganglioside, a glypican, a G protein-coupled receptor, a human papillomavirus protein, cancer/testis antigen, fibroblast activation protein, a member of the carbonic anhydrase family, a member of the carbohydrate antigen family, a Notch ligand, melanoma-associated chondroitin sulfate proteoglycan, glycoprotein A33, guanylate cyclase 2C and tumor-specific glycan,
wherein the targeting module and the at least one further targeting module comprise different target cell-binding domains, and identical tag-binding domains or tags.

In embodiments, the pharmaceutical composition is used in a method for treatment of cancer, infectious disease or autoimmune disease.

Another aspect of the invention is kit comprising
a) a targeting module comprising
   i) at least one CD19-binding domain comprising one of the sequences selected from SEQ ID No. 1 to SEQ ID No. 3 or a sequence identity of at least 95 % with one of the sequences selected from SEQ ID No. 1 to SEQ ID No. 3,
   ii) at least one CD20-binding domain comprising one of the sequences selected from SEQ ID No. 4 to SEQ ID No. 6 or with a sequence identity of at least 95 % with one of the sequences selected from SEQ ID No. 4 to SEQ ID No. 6, and
   iii) a tag-binding domain or a tag, and
b) a vector or a cell comprising a nucleotide sequence encoding a switchable chimeric antigen receptor, wherein the switchable chimeric antigen receptor comprises
   - a tag-binding domain or tag,
   - an extracellular hinge and a transmembrane domain and
   - a signal transduction domain,
wherein the tag-binding domain of the targeting module binds to the tag of the switchable chimeric antigen receptor or the tag of the targeting module binds to the tag-binding domain of the switchable chimeric antigen receptor.

In embodiments, the switchable CAR is a reversible universal chimeric antigen receptor (RevCAR). In embodiments, the kit comprises a vector or a cell comprising a nucleotide sequence encoding a RevCAR, comprising a tag, an extracellular hinge and a transmembrane domain and a signal transduction domain, wherein the tag-binding domain of the targeting module binds to the tag of the RevCAR.

In embodiments, the tag-binding domain or tag is present at the amino-terminal end of the polypeptide that comprises the switchable CAR. Advantageously, locating the tag-binding domain or the tag at the amino terminus permits unhampered access to the targeting module that is bound to the target cell. In embodiments, the RevCAR comprises a tag present at the amino-terminal end of the polypeptide that comprises the switchable CAR.

As used herein, the term "extracellular hinge and a transmembrane domain" refers to a flexible peptide sequence connected to the tag-binding domain or tag, which anchors the switchable CAR into the cell membrane of the cell and protrudes from the surface of the cell for optimal binding to its particular targeting module.

In embodiments, the extracellular hinge and a transmembrane domain is selected from the group comprising a hinge and transmembrane domain of human CD8α, CD28, ICOS (CD278), parts of a NK cell receptor, parts of the constant region of an antibody or mutants and combinations thereof. As used herein, the term "combinations thereof" refers to combinations of the different hinge and transmembrane domains.

Pinthus et al. and Cartellieri et al. describe the use of hinge and transmembrane domains of the human CD28 molecule in CARs (Pinthus et al. 2003, Cartellieri et al. 2016).

Milone et al. describes the use of hinge and transmembrane domains of human CD8α molecule in CARs (Milone et al. 2009).

Zhang et al. describe the use of hinge and transmembrane domains of NKG2D in CARs (Zhang et al. 2005).

Frigault et al. describes the use of hinge and transmembrane domains of parts of the constant region of immunoglobulin G1 (IgG) and the use of hinge domains of the constant region of IgG4 (Frigault et al. 2015).

Examples of combinations of the extracellular hinge and transmembrane domain are, but are not limited to, CD28 extracellular hinge and transmembrane domain, CD8alpha extracellular hinge and transmembrane domain, IgG1 or IgG4 constant regions combined with CD28 or CD137 transmembrane domain.

As used herein, the term "signal transduction domain" refers to a peptide sequence which transmits a signal into the cell by cross-linkage of the cell expressing the switchable CAR (effector cell) to a human cell surface protein or protein complex (target cell). Cross-linkage between effector and target cell is mediated by the targeting module.

In embodiments, the signal transduction domain comprises at least two signal transduction domains independently selected from a cytoplasmic region of a CD3 chain, CD28, CD137 (4 1BB), CD134 (OX40), CD278 (ICOS), DAP10, CD27, programmed cell death-1 (PD-1), cytotoxic T-lymphocyte antigen 4 (CTLA-4), DAP12, CD122 (interleukin-2 receptor β), CD132 (interleukin-2 receptor γ), CD127 (interleukin-7 receptor α), CD360 (interleukin-21 receptor), an activating Fc receptor and mutants thereof.

As used herein, the term "mutants" refers to proteins having at least 90 % sequence identity to the signal transduction domains, preferably at least 95 % sequence identity. Advantageously, the mutant transmits a signal into the cell by cross-linkage of the cell expressing the switchable CAR (effector cell) to a human cell surface protein or protein complex (target cell) in the same way as the named signal transduction domains.

In embodiments, mutants are truncated versions. As used herein, the term "truncated versions" refers to shortened proteins having at least 90 % sequence identity to the signal transduction domains, preferably at least 95 % sequence identity, more preferably having a chain length of at least 90 % and a sequence identity of 100 %, most preferably a chain length of at least 95 % and a sequence identity of 100 %. Advantageously, the truncated version has an activity of at least 80 %, preferably of at least 90 %, more preferably of at least 95 %; of the named signal transduction domains.

Hombach et al. describes the use of cytoplasmic regions of CD28 as signal transduction domain in CARs (Hombach et al. 2001). Guedan et al. describes the use of a mutant of cytoplasmic regions of CD28 as signal transduction domain (Guedan et al. 2020).

Finney et al. describes the use of cytoplasmic regions of CD137 (4-1BB) and of CD134 (OX40) as signal transduction domain (Finney et al. 2004).

Guedan et al. describes the use of cytoplasmic regions of CD278 (ICOS) as signal transduction domain (Guedan et al. 2018).

Zhang et al. describes the use of DAP10 as signal transduction domain (Zhang et al. 2005).

Fedorov et al. describes the use of programmed cell death 1 (PD-1) and of cytotoxic T-lymphocyte antigen 4 (CTLA-4) as signal transduction domain in CARs (Fedorov et al. 2013).

Gong et al. describes the use of cytoplasmic regions of CD3 chains, in particular the CD3ζ chain, as signal transduction domain in CARs (Gong et al. 1999).

Töpfer et al. describes the use of DAP12 as signal transduction domain in CARs (Töpfer et al. 2015).

Kagoya et al. describes the use of signaling chains or motifs derived from interleukin receptors as signal transduction domain in CARs (Kagoya et al. 2018).

Lamers et al. describes the use of activating Fc receptors, in particular the Fc epsilon receptor γ chain, as signal transduction domain (Lamers et al. 2004).

In embodiments, the signal transduction domain comprises at least two signal transduction domains independently selected from a cytoplasmic region of a CD3 chain, CD28, CD137 (4 1BB), CD134 (OX40), CD278 (ICOS), DAP10, CD27, programmed cell death-1 (PD-1), cytotoxic T-lymphocyte antigen 4 (CTLA-4), DAP12, CD122 (interleukin-2 receptor β), CD132 (interleukin-2 receptor γ), CD127 (interleukin-7 receptor α), CD360 (interleukin-21 receptor) and an activating Fc receptor.

In embodiments, the switchable chimeric antigen receptor comprises two, three or four signal transduction domains independently selected from a cytoplasmic region of a CD3 chain, CD28, CD137 (4-1BB), CD134 (OX40), CD278 (ICOS), DAP10, CD27, programmed cell death-1 (PD-1), cytotoxic T-lymphocyte antigen 4 (CTLA-4), DAP12, CD122 (interleukin-2 receptor β), CD132 (interleukin-2 receptor γ), CD127 (interleukin-7 receptor α), CD360 (interleukin-21 receptor) and an activating Fc receptor.

In further embodiments, the switchable CAR comprises a fourth domain, wherein the fourth domain is a short peptide linker in the extracellular portion of the receptor that may serve to detect the chimeric antigen receptor on the cell surface or stimulate the chimeric antigen receptor T cell. Advantageously, the switchable CAR engrafted cells with the fourth domain can be specifically stimulated to proliferate preferentially and persist longer compared to non-engrafted cells either in *vitro* or in *vivo.* Further advantageously, the fourth domain may be also used to purify switchable CAR engrafted cells from mixed cell populations or to dampen switchable CAR engrafted cell-mediated immune response and to eliminate switchable CAR engrafted cells *in vivo.* In embodiments, the fourth domain comprises at least one linear epitope, preferably E7B6 according to SEQ ID No. 23 or SEQ ID No. 24.

In embodiments, the fourth domain is located in between the tag-binding domain or the tag and the extracellular hinge domain or an integral part of the extracellular hinge domain.

In further embodiments, the switchable CAR comprises a signal peptide. Advantageously, the signal peptide allows for expression on the cell surface of an effector cell. In embodiments, the signal peptide is located at the N-terminus of the switchable CAR nucleotide sequence in front of the tag-binding domain or the tag. In embodiments, the signal peptide targets proteins to the secretory pathway either co-translationally or post-translationally. In embodiments, the signal peptide is a human signal peptide selected from CD8α, CSF2Rα, CD28, IL-2, lysozyme C, a heavy chain of an antibody, a light chain of an antibody or a part of a light chain of an antibody.

In embodiments, the switchable chimeric antigen receptor comprises an amino acid sequence according to SEQ ID No. 77 to SEQ ID No. 107.

In embodiments, the nucleotide sequence encoding a switchable chimeric antigen receptor comprises a nucleotide sequence according to SEQ ID No. 108 to SEQ ID No. 137.

According to the invention, the vector and/or cell are isolated.

In embodiments, the vector is a plasmid, an artificial chromosome, linearized DNA or RNA, a virus particle or another vector that contains an expression cassette that is incorporated stably into the genome of a host cell or host organism.

In embodiments, the cell is selected from immune cells, preferably with cytolytic, phagocytic or immunosuppressive activity, such as T cells, Natural Killer (NK) cells and macrophages. In embodiments, the cell is selected from T cells, including alpha/beta and gamma/delta T cells or subpopulations of T cells like stem-cell memory T cells or central memory T cells, cytotoxic T cells or NK cells.

In embodiments, the vector or cell further comprises an inducible expression system. In some embodiments, the inducible expression system is based on a prokaryotic operon, including, but not limited to, the lac operon, transposon Tn₁₀ or tetracycline operon. In other embodiments, the inducible expression system is based on components of a eukaryotic signaling pathway, including, but not limited to, expression systems based on a steroid receptor, an estrogen receptor, progesterone or metallothionein.

In embodiments, the inducible expression system induces the transcription of the nucleotide sequence encoding a switchable CAR and/or a nucleotide sequence encoding a targeting module according to the invention, preferably the inducible expression system induces the transcription of the nucleotide sequence encoding a targeting module according to the invention.

In embodiments, the kit further comprises at least one further targeting module,
wherein the at least one further targeting module comprises at least one target cell-binding domain and a tag-binding domain or a tag,
wherein the at least one target cell-binding domain is an antibody, antibody fragment, a protein, a peptide or a low molecular weight organic ligand that binds to surface antigens selected from the group comprising CD2, CD3, CD4, CD5, CD7, CD8, CD10, CD15, CD22, CD23, CD25, CD30, CD33, CD38, CD44, CD44v6, CD52, CD66a, CD66b, CD66c, CD66d, CD66e, CD66f, CD90, CD99, CD123, CD133, CD135, CD150, CD181, CD182, CD184, CD223, CD229, CD269, CD273, CD274, CD276, CD279, CD319, CD366, CD371, cytokine receptors, CXCR4, c-Met, mesothelin, a member of the epidermal growth factor receptor family or a mutant thereof, a member of the tumor necrosis factor receptor superfamily, a claudin, ephrin, an ephrin receptor, a fucosyl transferase, a prostate specific antigen, an embryonic antigen, a member of the vascular endothelia growth factor family, epithelial cell adhesion molecule, alpha-fetoprotein, a member of the intercellular adhesion molecule family, a C-type lectin, an integrin, a member of the mucin protein family, a follicle-stimulating hormone receptor, a high molecular weight-melanoma associated antigen, a folate binding protein, a folate receptor, a somatostatin receptor, a ligand of the NKG2D receptor, a member of the epithelia glycoprotein family, a diasialogangliosde, a glypican, a G protein-coupled receptor, a human papillomavirus protein, cancer/testis antigen, fibroblast activation protein, member of the carbonic anhydrase family, member of the carbohydrate antigen family, Notch ligand, melanoma-associated chondroitin sulfate proteoglycan, glycoprotein A33, guanylate cyclase 2C and tumor-specific glycan,
wherein the targeting module and the at least one further targeting module comprise different target cell-binding domains, and identical tag-binding domains or tags.

In embodiments, the kit comprises one to three targeting modules, preferably one targeting module according to the invention and one or two further targeting modules.

In embodiments, the targeting module and/or the cell comprising a nucleotide sequence encoding a switchable chimeric antigen receptor are in the form of a pharmaceutical composition.

In embodiments, the kit is used in the treatment of cancer, infectious disease or autoimmune disease.

In embodiments, the kit is used for the manufacture of a medicament for the treatment of cancer, infectious disease or autoimmune disease.

In embodiments, the kit is used in a method for treatment of cancer, infectious disease or autoimmune disease.

In embodiments, the method for treatment of cancer, infectious disease or autoimmune disease comprises the administration of a targeting module, a cell or a vector, a pharmaceutical composition or a kit according to the invention to a subject in need thereof, preferably a mammal, more preferable a human, having cancer, an infectious or an autoimmune disease. For therapeutic applications, a sterile pharmaceutical composition according to the invention or a sterile kit according to the invention, comprising a pharmacologically effective quantity of targeting module according to the invention and a vector or a cell comprising a nucleotide sequence encoding a switchable chimeric antigen receptor, is administered to a subject in order to treat the aforementioned illnesses.

In some embodiments, the method for treatment of cancer, infectious or autoimmune disease, comprises the following steps:
a) administering to a mammal an effective amount of a targeting module according to the invention and
b) administering to the mammal an effective amount of a vector or a cell comprising a nucleotide sequence encoding a switchable chimeric antigen receptor,
   wherein the switchable chimeric antigen receptor comprises
      - a tag-binding domain or tag,
      - an extracellular hinge and a transmembrane domain and
      - a signal transduction domain,
   wherein the tag-binding domain of the targeting module binds to the tag of the switchable chimeric antigen receptor or the tag of the targeting module binds to the tag-binding domain of the switchable chimeric antigen receptor,
wherein the targeting module is administered to a mammal prior to, concurrent with or after the administration of the vector or cell.

The invention is not limited to the specifically described combinations of features but may also be defined by any other combination of specific features of all the individual features disclosed as a whole, provided that the individual features are not mutually exclusive, or a specific combination of individual features is not explicitly excluded.

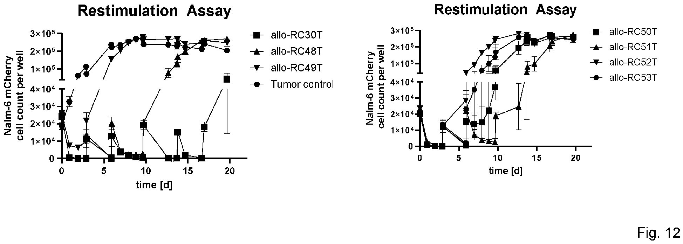

The following detailed description of exemplary embodiments of the invention is presented to enable any person skilled in the art to make and use the disclosed subject matter in the context of one or more implementations. Various modifications to the disclosed implementations will be readily apparent to those skilled in the art, and the general principles defined herein may be applied to other implementations and applications without departing from scope of the disclosure. Thus, the present disclosure is not intended to be limited to the described or illustrated implementations but is to be accorded the widest scope consistent with the principles and features disclosed herein.

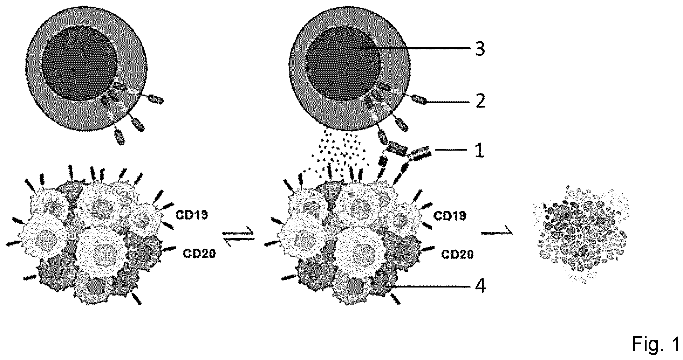

**Fig. 1** shows a schematic illustration of the mode of action of engineered immune cell in combination with a CD19- and CD20-specific targeting module (TM) together forming the active drug. A switchable CAR expressing immune cell carries the tag-binding domain or tag on the cell surface. When ligands (targeting module) binding to the tag-binding domain or tag are not present within the human body immune cells remain in an off-mode (left). Antigen-specificity of switchable CAR immune cell is provided via the soluble targeting module (TM) with exclusive specificity for the target antigens CD19 and CD20, consisting of at least two target cell-binding domains (CD19-binding domain and CD20-binding domain) and a tag or tag-binding domain specific for the tag-binding domain or tag of the switchable CAR. Cross-linking of switchable CAR-immune cell and the target antigen-expressing tumor cell by TMs activates switchable CAR-immune cell effector functions and subsequently killing of the tumor cells (right).

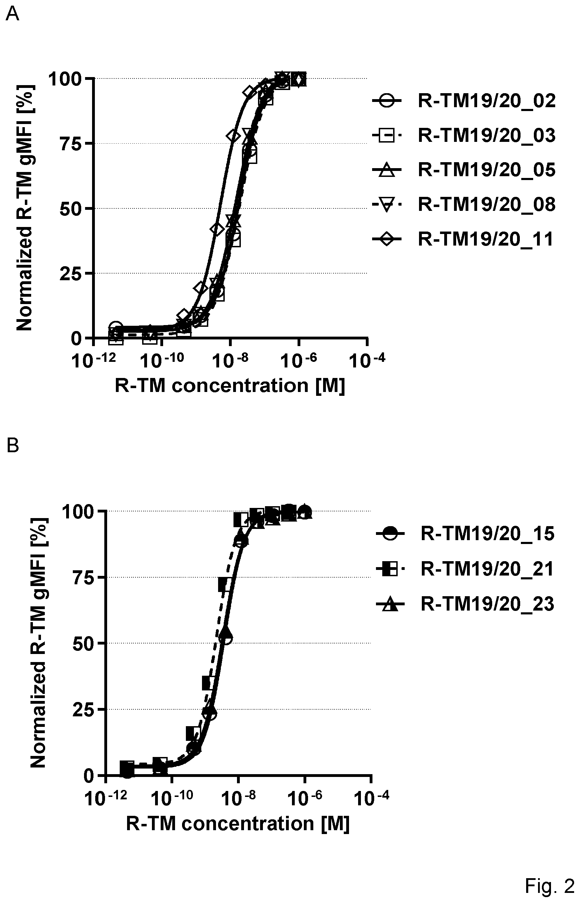

**Fig. 2** shows a cellular binding assay of different targeting modules according to the invention on CD19-positive OCI-AML3-CD19 cells: normalized gMFls of OCI-AML3-CD19 cells binding to **A)** CD19 VHH-based CD19-CD20-targeting modules according to the invention and **B)** CD19 scFv-based CD19-CD20-targeting modules according to the invention. Error bars represent the standard error of the mean from biological triplicates.

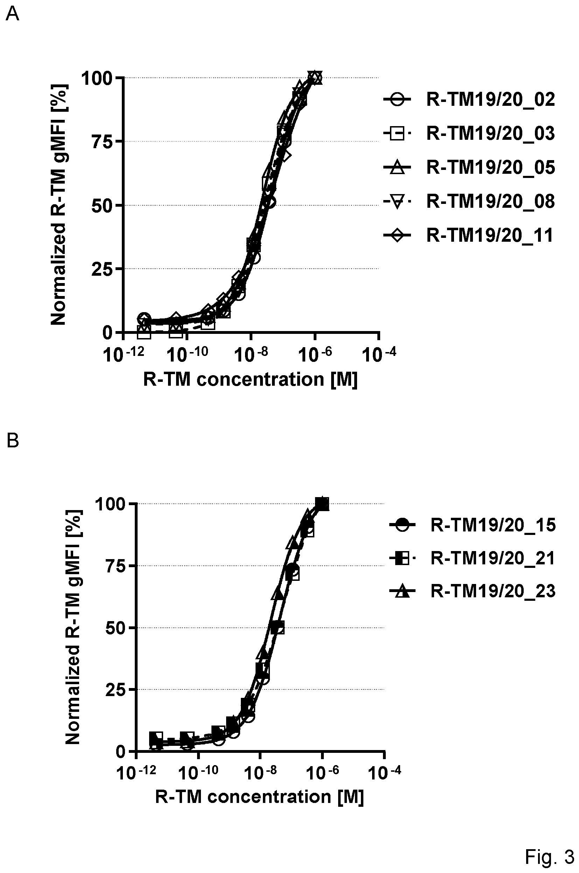

**Fig. 3** shows a cellular binding assay of different targeting modules according to the invention on CD20-positive OCI-AML3-CD20 cells: normalized gMFls of OCI-AML3-CD20 cells binding to **A)** CD19 VHH-based CD19-CD20-targeting modules according to the invention and **B)** CD19 scFv-based CD19-CD20-targeting modules according to the invention. Error bars represent the standard error of the mean from biological triplicates.

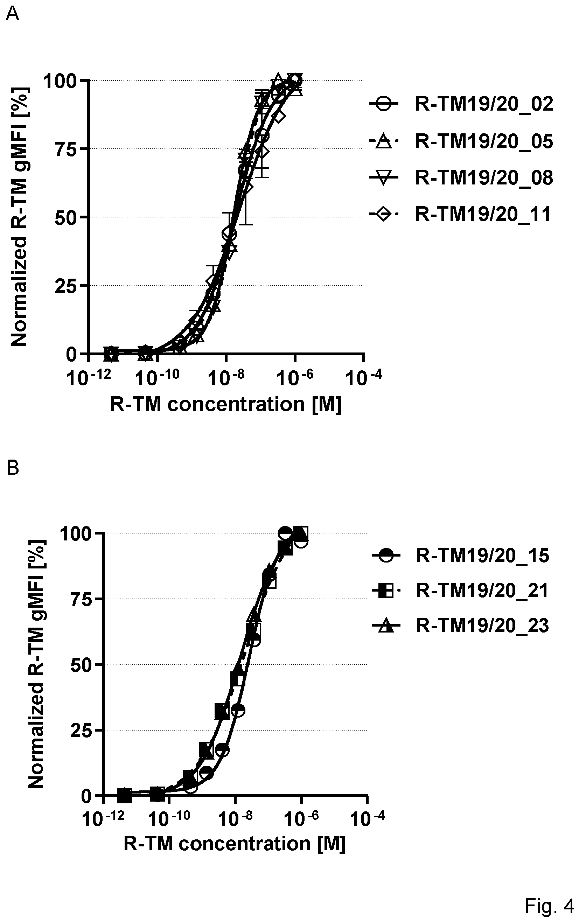

**Fig. 4** shows a cellular binding assay of different targeting modules according to the invention on CD19/CD20-positive NALM-6-CD20 cells: normalized gMFls of NALM-6-CD20 cells binding to **A)** CD19 VHH-based CD19-CD20-targeting modules according to the invention and **B)** CD19 scFv-based CD19-CD20-targeting modules according to the invention. Error bars represent the standard error of the mean from biological triplicates.

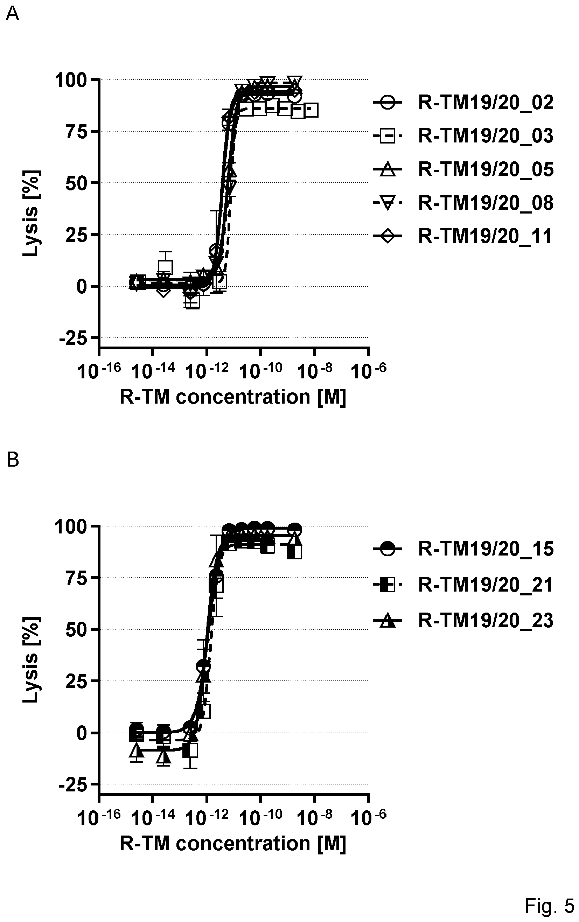

**Fig. 5** shows a cytotoxicity assay with targeting module (R-TM) titration: Co-culture of RevCAR-T cells and CD19-positive OCI-AML3-CD19 target cells at effector cell (E):target cell (T) ratio of 1:2 with **A)** CD19 VHH-based CD19-CD20-targeting modules serial dilutions and **B)** CD19 scFv-based CD19-CD20-targeting modules serial dilutions. Target cell counts were determined via FACS at 48 hours after adding targeting module in reference to OCI-AML3-CD19 cell counts in target cell-only conditions (no targeting module (R-TM), no RevCAR-T cells (RC01)). Each data point represents the mean of three biological replicates (different T cell donors). Error bars represent the standard error of the mean.

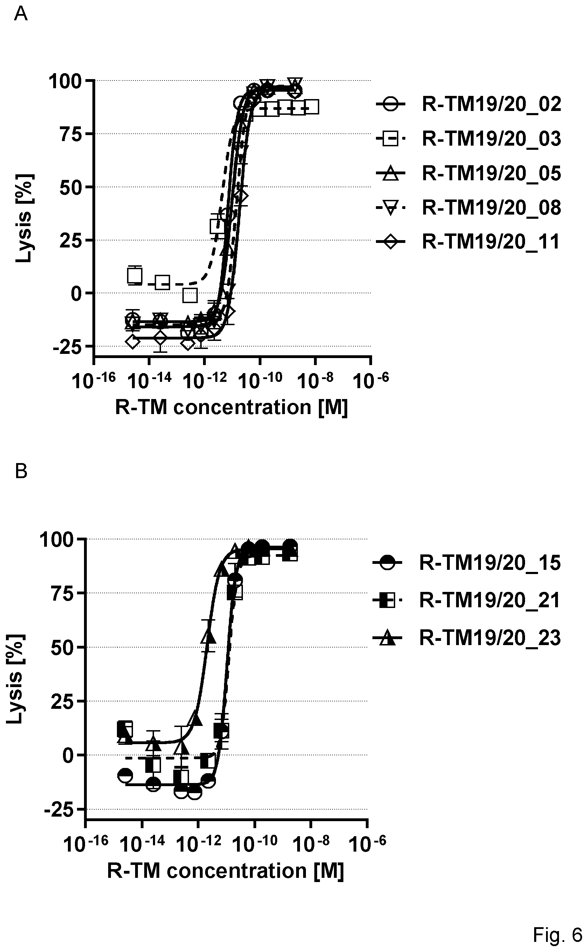

**Fig. 6** shows a cytotoxicity assay with targeting module (R-TM) titration: Co-culture of RevCAR-T cells and CD20-positive OCI-AML3-CD20 target cells at E:T ratio of 1:2 with **A)** CD19 VHH-based CD19-CD20-targeting modules serial dilutions and **B)** CD19 scFv-based CD19-CD20-targeting modules serial dilutions. Target cell counts were determined via FACS at 48 hours after adding targeting module in reference to OCI-AML3-CD19 cell counts in target cell-only conditions (no R-TM, no RC01). Each data point represents the mean of three biological replicates (different T cell donors). Error bars represent the standard error of the mean.

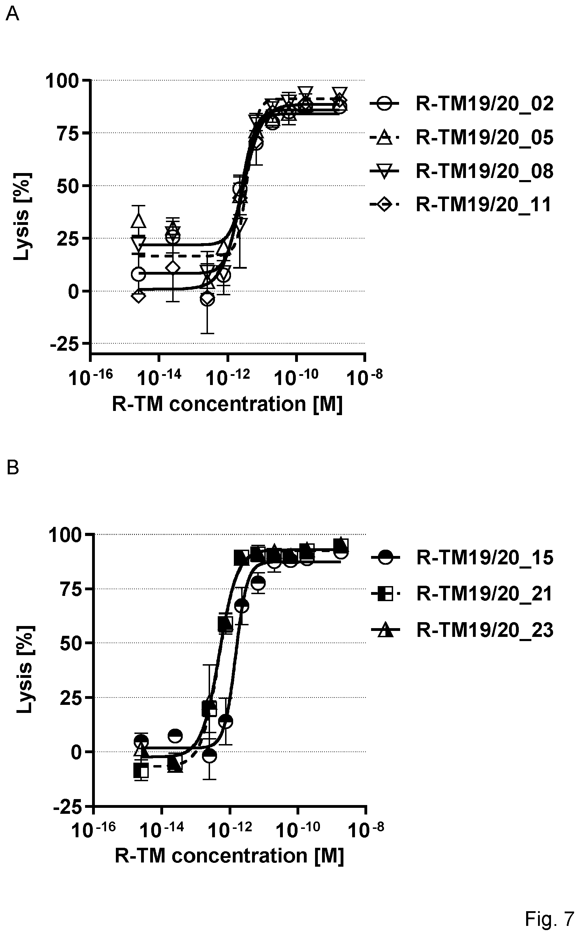

**Fig. 7** shows a cytotoxicity assay with targeting module (R-TM) titration: Co-culture of RevCAR-T cells and CD19/CD20-positive positive NALM-6-CD20 target cells at E:T ratio of 1:2 with **A)** CD19 VHH-based CD19-CD20-targeting modules serial dilutions and **B)** CD19 scFv-based CD19-CD20-targeting modules serial dilutions. Target cell counts were determined via FACS at 48 hours after adding targeting module in reference to OCI-AML3-CD19 cell counts in target cell-only conditions (no R-TM, no RC01). Each data point represents the mean of three biological replicates (different T cell donors). Error bars represent the standard error of the mean.

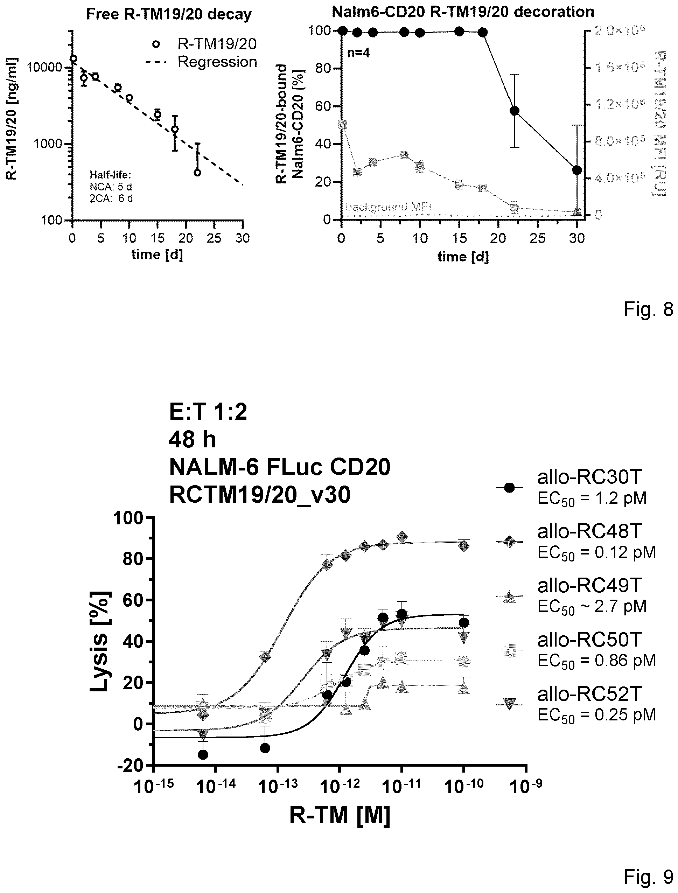

**Fig. 8** shows pharmacokinetic of the targeting module according to the invention (R-TM19/20) and bioavailability in Nalm6-CD20 engrafted NSG mice. Left: Concentration decay over time of targeting module after a single bolus intravenous injection of 1 µg/g in tumor-bearing NSG mice as determined by ELISA. Right: Nalm6-CD20 cell decoration with targeting module detected with anti-Fc mab by flow cytometry in bone marrow samples. Mice were pre-engrafted with 5·10⁵ Nalm6-CD20 cells 7 days prior to bolus injection of targeting module.
**Fig. 9** shows in vitro potency assay results for allogeneic switchable CAR (allo-RevCAR) T cells against Nalm6-CD20. Co-culture assay of allo-RevCAR T cell products with target cell line Nalm6-CD20 in presence of varying concentrations of targeting module according to the invention (R-TM19/20). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the sensitivity of allo-RevCAR T cell products to the targeting module used.

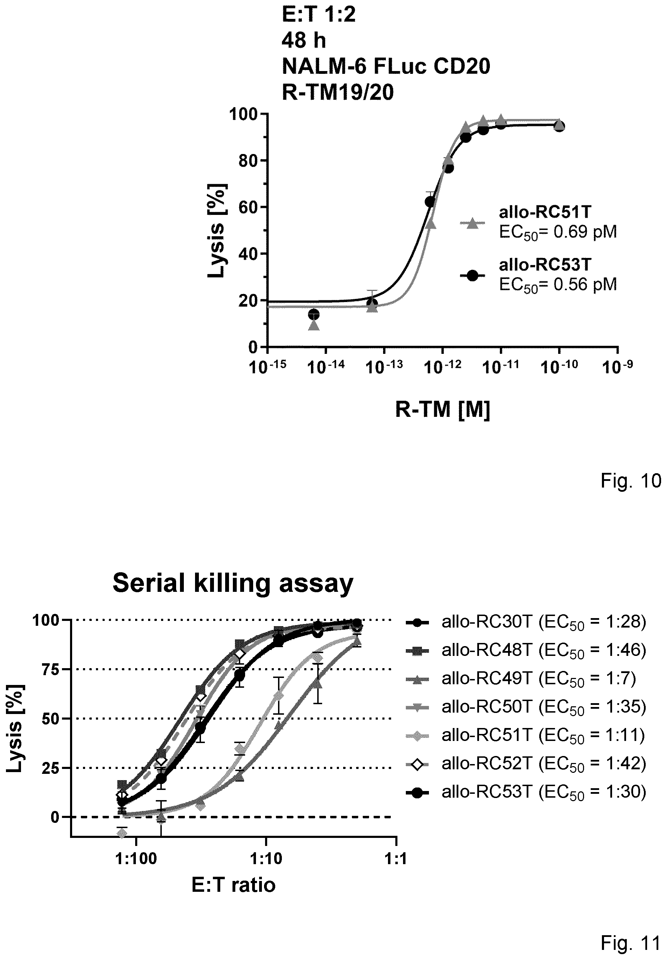

**Fig. 10** shows in vitro potency assay results for switchable CAR (allo-RevCAR) T cells against Nalm6-CD20. Co-culture assay of allo-RevCAR T cell products with target cell line Nalm6-CD20 in presence of varying concentrations of targeting module according to the invention (R-TM19/20). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the sensitivity of allo-RevCAR T cell products to the targeting module used.
**Fig. 11** shows results of a serial killing assay results of allogeneic switchable CAR (allo-RevCAR) T cells against Nalm6-CD20 cells. Co-culture assay of allo-RevCAR T cell products with target cell line Nalm6-CD20 in presence of 1 nM targeting module according to the invention (R-TM19/20). Target cells were stained beforehand with the proliferation dye efluor 450. After 48 hrs, viable target cell count is determined by flow cytometry, dead cells are discriminated with propidium iodide and specific lysis is calculated (relative to control without targeting module).
Mean of three replicates is plotted and fitted with a 4-parameter logistic regression. Calculated EC50 values represent the capacity of RevCAR T cell products to kill multiple target cells. **Fig. 12** shows results of a long-term restimulation assay results of allogeneic switchable CAR (allo-RevCAR) T cells against Nalm6 cells. Co-culture assay of allo-RevCAR T cell products with target cell line Nalm6 (mcherry positive) in presence of 0.2 nM targeting module according to the invention (R-TM19/20). Every 3 to 4 days 2 10" fresh target cells are added. Mcherry positive target cell counts are determined by fluorescence microscopy (Incucyte S3) twice a day over three weeks. Mean and standard deviation of four replicates is plotted.

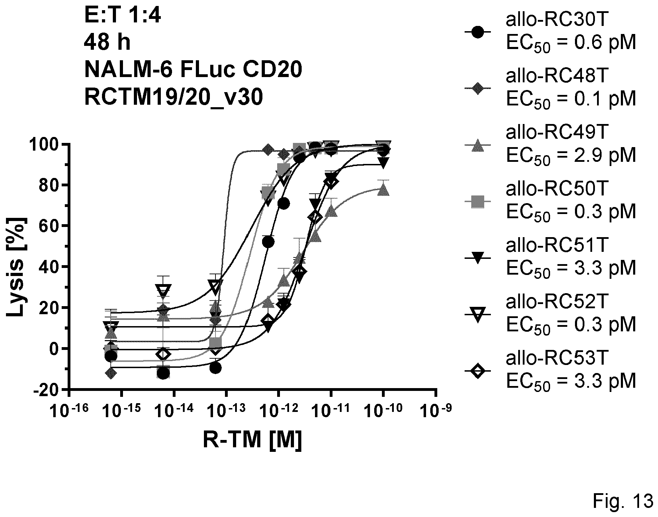

**Fig. 13** shows in vitro potency assay results for allogeneic switchable CAR (allo-RevCAR) T cells against Nalm6-CD20. Co-culture assay of allo-RevCAR T cell products with target cell line Nalm6-CD20 in presence of varying concentrations of targeting module according to the invention (R-TM19/20). Target cells were stained beforehand with the proliferation dye efluor 450. After 48 hrs, viable target cell count is determined by flow cytometry, dead cells are discriminated with propidium iodide and specific lysis is calculated (relative to control without R-TM). Mean of three replicates is plotted and fitted with a 4-parameter logistic regression. Calculated EC50 values represent the sensitivity of RevCAR T cell products to the targeting module used.

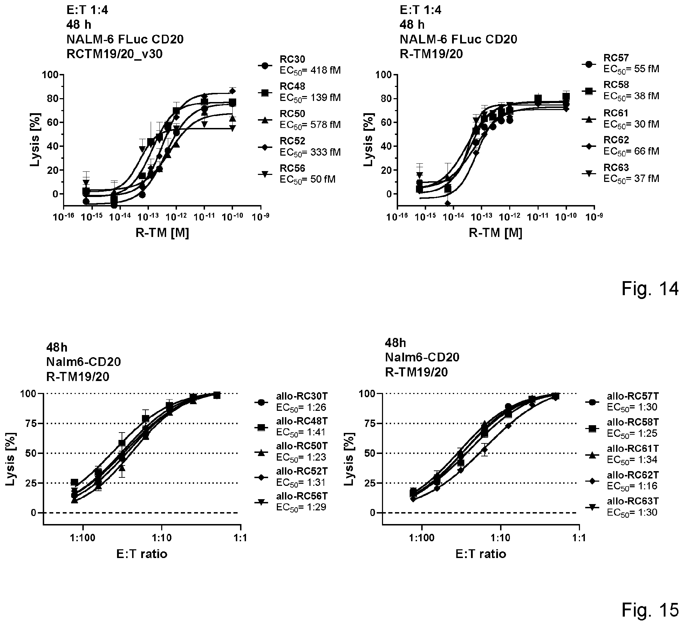

**Fig. 14** shows in vitro potency assay results for allogeneic switchable CAR (allo-RevCAR) T cells against Nalm6-CD20. Co-culture assay of allo-RevCAR T cell products with target cell line Nalm6-CD20 in presence of varying concentrations of targeting module according to the invention (R-TM19/20). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the sensitivity of RevCAR T cell products to the targeting module used.
**Fig. 15** shows serial killing assay results of allogeneic switchable CAR (allo-RevCAR) T cells against Nalm6-CD20 cells. Co-culture assay of allo-RevCAR T products with target cell line Nalm6-CD20 in presence of 1nM targeting module according to the invention (R-TM19/20). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the capacity of RevCAR T cell products to kill multiple target cells.

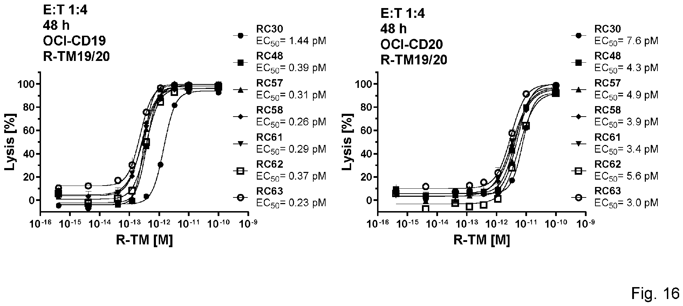

**Fig. 16** shows in vitro potency assay results for allogeneic switchable CAR (allo-RevCAR) T cells against Oci-AML-CD19 and Oci-AML-CD20 cells. Co-culture assay of allo-RevCAR T cell products with target cell line Oci-AML-CD19 and Oci-AML-CD20 in presence of varying concentrations of targeting module according to the invention (R-TM19/20). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the sensitivity of RevCAR T cell products to the targeting module used.

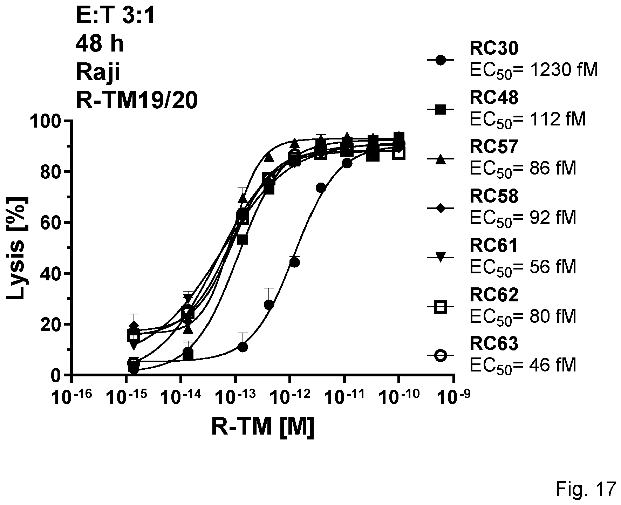

**Fig. 17** shows in vitro potency assay results for allogeneic switchable CAR (allo-RevCAR) T cells against Raji lymphoma cells. Co-culture assay of allo-RevCAR T cell products with target cell line Raji in presence of varying concentrations of targeting module according to the invention (R-TM19/20). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the sensitivity of RevCAR T cell products to the targeting module used.

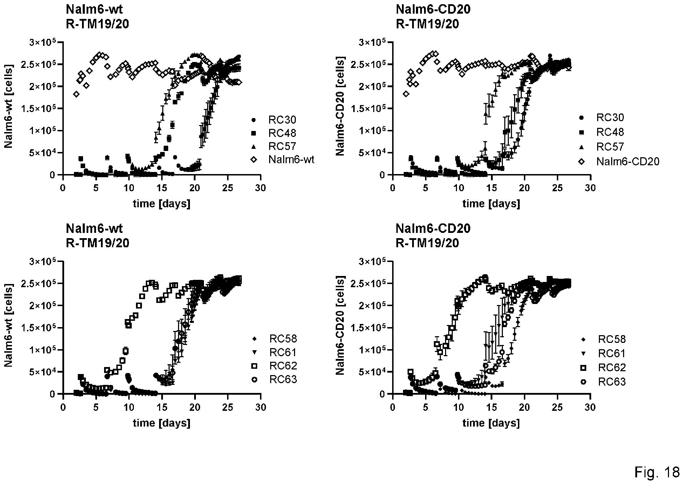

**Fig. 18** shows in long-term restimulation assay results of allogeneic switchable CAR (allo-RevCAR) T cells against Nalm6 and Nalm6-CD20 cells. Co-culture assay of allo-RevCAR T cell products with target cell line Nalm6 (mcherry positive) and Nalm6-CD20 (mcherry positive) in presence of 0.2 nM targeting module according to the invention (R-TM19/20). Every 3 to 4 days 2 10" fresh target cells are added. Mcherry positive target cell counts are determined by fluorescence microscopy (Incucyte S3) twice a day over three weeks. Mean and standard deviation of three replicates is plotted.

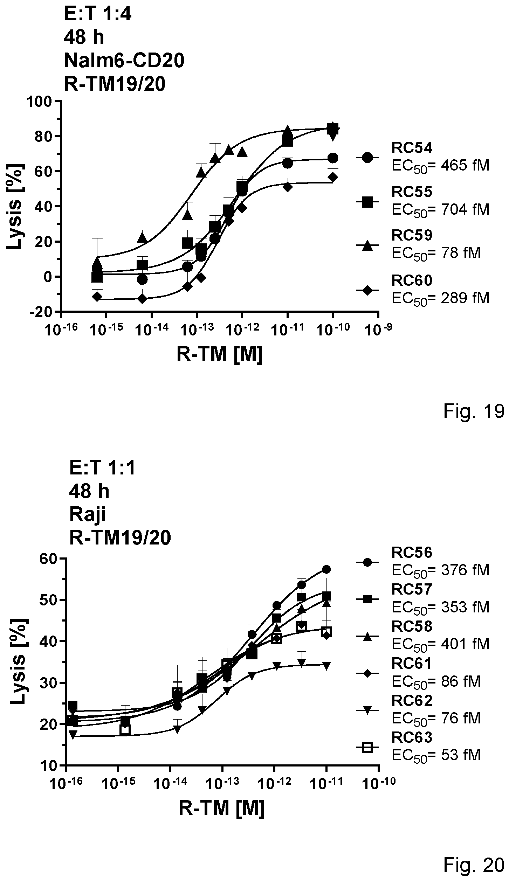

**Fig. 19** shows in vitro potency assay results for allogeneic switchable CAR (allo-RevCAR) T cells against Nalm6-CD20 cells. Co-culture assay of allo-RevCAR T cell products with target cell line Nalm6-CD20 in presence of varying concentrations of targeting module according to the invention (R-TM19/20). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the sensitivity of RevCAR T cell products to the targeting module used.
**Fig. 20** shows in vitro potency assay results for allogeneic switchable CAR (allo-RevCAR) T cells against Raji lymphoma cells. Co-culture assay of allo-RevCAR T cell products with target cell line Raji in presence of varying concentrations of targeting module according to the invention (R-TM19/20). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the sensitivity of RevCAR T cell products to the targeting module used.

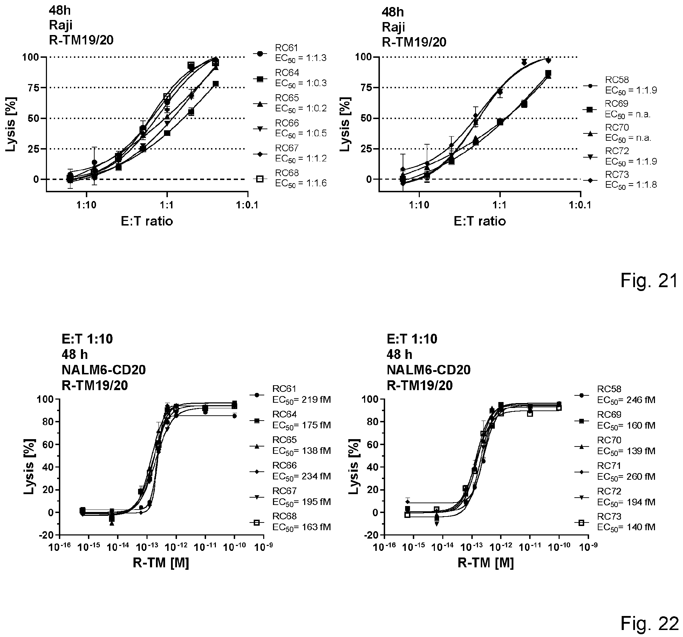

**Fig. 21** shows serial killing assay of allogeneic switchable CAR (allo-RevCAR) T cells against Raji lymphoma cells. Co-culture assay of allo-RevCAR T cell products with target cell line Raji in presence of 0.1 nM targeting module according to the invention (R-TM19/20). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the capacity of RevCAR T cell products to kill multiple target cells.
**Fig. 22** shows in vitro potency assay results for allogeneic switchable CAR (allo-RevCAR) T against Nalm6-CD20 cells. Co-culture assay of allo-RevCAR T cell products with target cell line Nalm6-CD20 in presence of varying concentrations of targeting module according to the invention (R-TM19/20). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the sensitivity of RevCAR T cell products to the targeting module used.

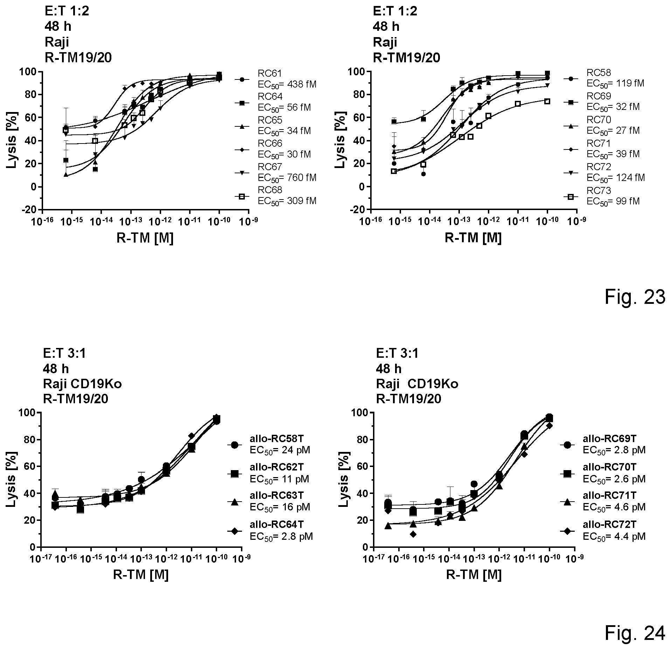

**Fig. 23** shows in vitro potency assay results for allogeneic switchable CAR (allo-RevCAR) T cells against Raji lymphoma cells. Co-culture assay of allo-RevCAR T cell products with target cell line Raji in presence of varying concentrations of targeting module according to the invention (R-TM19/20). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the sensitivity of RevCAR T cell products to the targeting module used.
**Fig. 24** shows in vitro potency assay results for allogeneic switchable CAR (allo-RevCAR) T cells against Raji CD19Ko lymphoma cells. Co-culture assay of allo-RevCAR T cell products with target cell line Raji CD19Ko (negative for CD19) in presence of varying concentrations of targeting module according to the invention (R-TM19/20). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the sensitivity of RevCAR T cell products to the targeting module used.

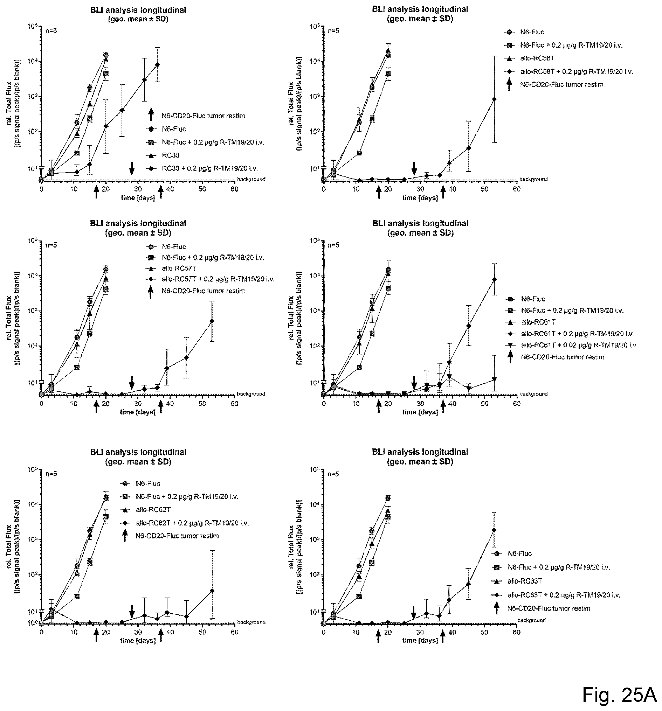

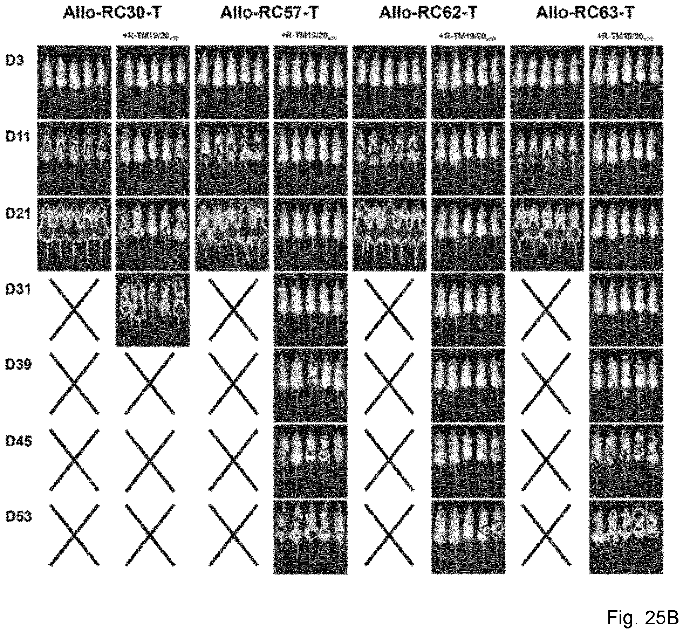

**Fig. 25A** **and** **25B** show in vivo efficacy results of allogeneic switchable CAR (allo-RevCAR) T cells against a B-ALL Nalm6-CD20 tumor. Evaluation of tumor control and tumor regression capacity of allo-RevCAR T cells in Nalm6-CD20-Fluc xenograft mice. Mice received 5·10⁵ Nalm6-CD20-Fluc cells 7 days prior to transplantation of 15·10⁶ allogeneic T cells (total). Therapy was initiated one day before mice received T cells with a bolus injection of 0.2 µg/g or 0.02 µg/g targeting module according to the invention (R-TM19/20) intravenously, respectively. Mice which received allo-RevCAR T cells and targeting module were rechallenged with tumor cells at day 17 (1·10⁶ cells), day 28 (2·10⁶ cells) and day 37 (5·10⁶ cells). Normalized radiance for bioluminescent imaging (BLI) is ranging from 5·10⁴ to 1·10⁷ [p/sec/cm²sr].

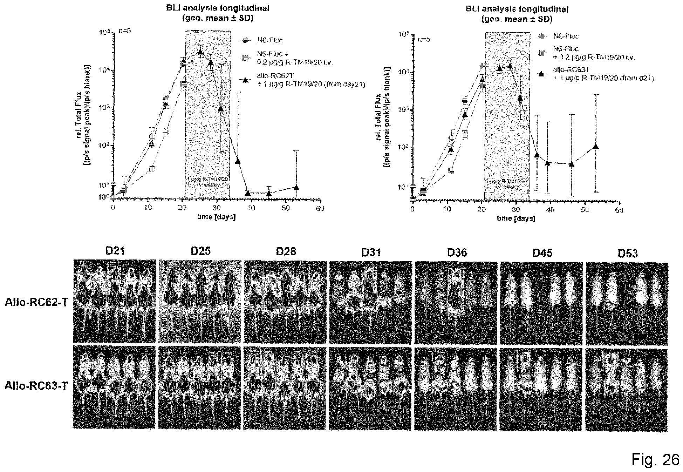

**Fig. 26** shows in vivo efficacy results of allogeneic switchable CAR (allo-RevCAR) T cells against high burden B-ALL Nalm6-CD20 tumor in late-stage mice. Evaluation of tumor control and tumor regression capacity of allo-RC62T and RC63T cells in late-stage high tumor burden Nalm6-CD20-Fluc xenograft model. Mice received 5·10⁵ Nalm6-CD20-Fluc cells 7 days prior to transplantation of 15·10⁶ allo-RevCAR T cells (total). Therapy was initiated at day 21 after tumor application with intravenous bolus injections of targeting module according to the invention (R-TM19/20) in doses of 1.0 µg/g (day 21, day 28 and day 35) and 0.2 µg/g (day 44). Normalized radiance for BLI is ranging from 5·10⁴ to 1·10⁷ [p/sec/cm²sr].

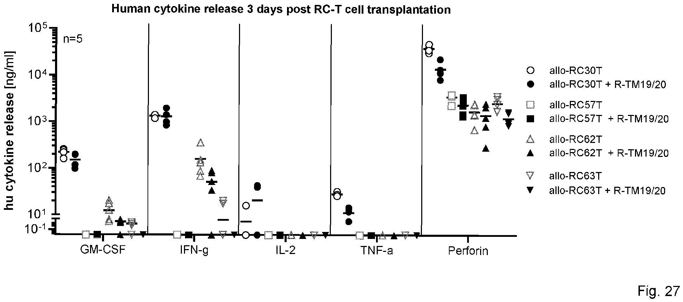

**Fig. 27** shows cytokine release of human allogeneic switchable CAR (allo-RevCAR) T cells in mice. Quantification of human cytokines (GM-CSF, IFN-γ, IL-2, TNF-α and Perforin) from peripheral blood of Nalm6-CD20 xenograft mice three days after allo-RevCAR T cell transplantation and initiation of therapy with targeting module according to the invention (R-TM19/20). (MACSPlex Cytotoxic T/NK Cell kit,from Miltenyi, Germany).

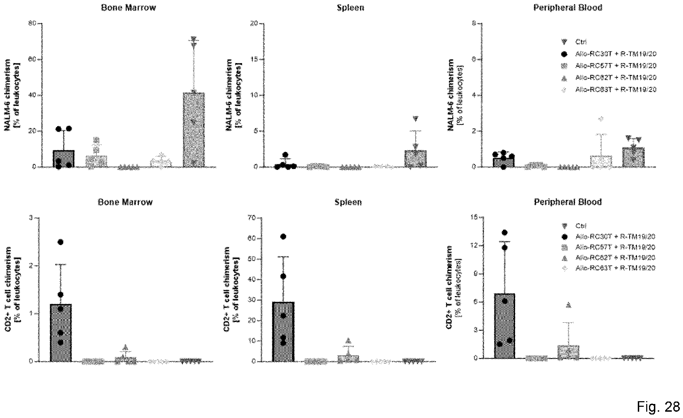

**Fig. 28** shows tumor and T cell chimerism determined at individual end points via flow cytometry. Mice received 5·10⁵ Nalm6-CD20-Fluc cells 7 days prior to transplantation of 15·10⁶ allogeneic switchable CAR (allo-RevCAR) T cells (total). Therapy was initiated one day before mice received allo-RevCAR T cells with a bolus injection of 0.2 µg/g or 0.02µg/g targeting module according to the invention (R-TM19/20) intravenously, respectively. Mice which received allo-RevCAR T cells and targeting module were rechallenged with tumor cells at day 17 (1·10⁶ cells), day 28 (2·10⁶ cells) and day 37 (5·10⁶ cells).

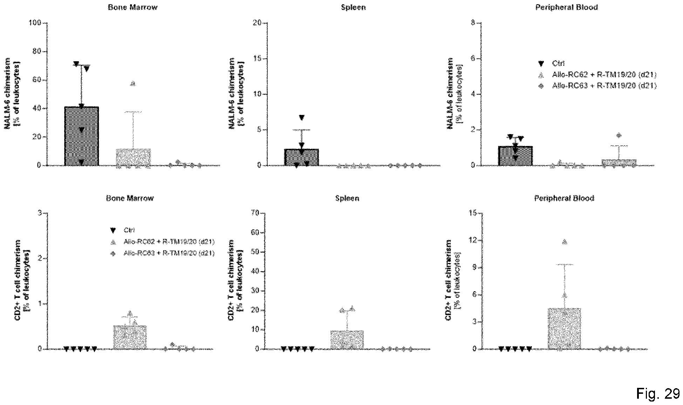

**Fig. 29** shows tumor and T cell chimerism determined at individual end points via flow cytometry in high tumor burden mice. Mice received 5·10⁵ Nalm6-CD20-Fluc cells 7 days prior to transplantation of 15·10⁶ allogeneic switchable CAR (allo-RevCAR) T cells (total). Therapy was initiated at day 21 after tumor application with intravenous bolus injections of targeting module according to the invention (R-TM19/20) in doses of 1.0 µg/g (day 21, day 28 and day 35) and 0.2 µg/g (day 44).

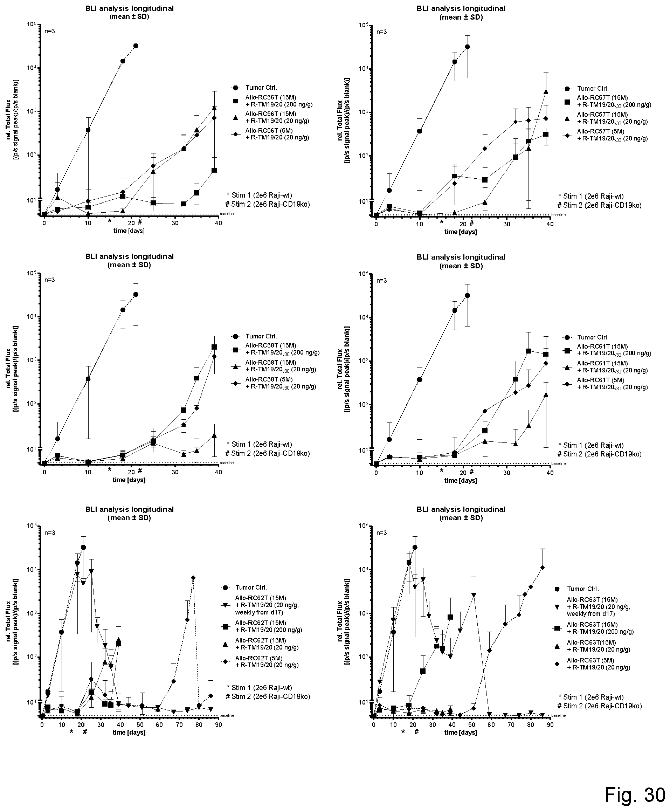

**Fig. 30** shows in vivo efficacy of allogeneic switchable CAR (allo-RevCAR) T cells against Raji lymphoma. Evaluation of tumor control and tumor regression capacity of allo-RevCAR T cells against Raji-Fluc in NSG mice. NSG mice received 5·10⁵ Raji-Fluc cells intravenously 7 days prior to transplantation of 15·10⁶ or 5·10⁶ allo-RevCAR T cells (total). Therapy was initiated 3 days prior to T cell transplantation with 0.2 µg/g or 0.02 µg/g targeting module according to the invention (R-TM19/20) intravenously and biweekly repeated. Mice which received allo-RevCAR T cells and targeting module were rechallenged once with 2·10⁶ Raji-Fluc tumor cells at day 15 and 2·10⁶ Raji CD19KO tumor cells at days 22. For groups of allo-RC62T cells and allo-RC63T cells which initially received no targeting module, therapy started at day 17 with 0.02 µg/g targeting module intravenously and was repeated weekly, here no tumor rechallenge was applied.

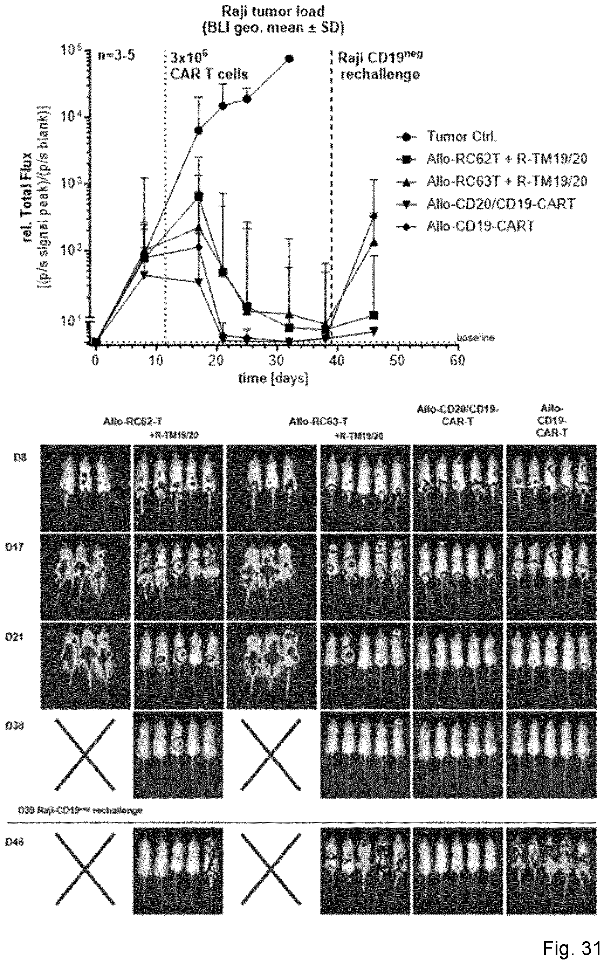

**Fig. 31** shows in vivo efficacy of allogeneic switchable CAR (allo-RevCAR) T cells against B-cell lymphoma. Evaluation of tumor control and tumor regression capacity of allo-RevCAR T cells against Raji-Fluc in NSG mice. NSG mice were engrafted with 5·10⁵ Raji-Fluc cells intravenously 11 days prior to receiving 3·10⁶ CAR T cells intravenously. One day before allo-RevCAR T cells got transplanted, therapy with 0.05 µg/g targeting module according to the invention (R-TM19/20) intravenously started and was repeated weekly. All targeting module treated animals got rechallenged with 2·10⁶ CD19neg Raji cells at day 39. Normalized radiance for BLI is ranging from 5·10⁴ to 1·10⁷ [p/sec/cm²/sr].

### Design of targeting modules according to the invention

The targeting module is a soluble, recombinant fusion protein comprising two antibody-derived binding domains and a tag. One selectively binds to the target antigen CD19 and one to the target antigen CD20, the other recognizes the tag-binding domain presented on the RevCAR expressing cells (epitope E5B9 from the human La protein). Thus, the TM functions as a bridging module between RevCAR-T and a CD19 and CD20-expressing target cancer cell (Fig. 1).

**Tab. 1 Design of different targeting modules according to the invention (SEQ ID No.44 to SEQ ID No. 76)**

| **Targeting module** | **Design** | | |
|---|---|---|---|
| | **1. chain** | **2. chain** | **3. chain** |
| R-TM-19/20_2 | SEQ ID No. 47 | SEQ ID No. 45 | SEQ ID No. 51 |
| R-TM-19/20_3 | SEQ ID No. 47 | SEQ ID No. 45 | SEQ ID No. 53 |
| R-TM-19/20_5 | SEQ ID No. 47 | SEQ ID No. 45 | SEQ ID No. 57 |
| R-TM-19/20_8 | SEQ ID No. 76 | SEQ ID No. 45 | SEQ ID No. 63 |
| R-TM-19/20_11 | SEQ ID No. 76 | SEQ ID No. 45 | SEQ ID No. 69 |
| R-TM-19/20_15 | SEQ ID No. 73 | SEQ ID No. 45 | SEQ ID No. 57 |
| R-TM-19/20_21 | SEQ ID No. 73 | SEQ ID No. 45 | SEQ ID No. 51 |
| R-TM-19/20_23 | SEQ ID No. 73 | SEQ ID No. 45 | SEQ ID No. 53 |

### Preparation of the targeting module

Expression was mediated from an ExpiCHO cell line in ExpiCHO expression medium in 2000 ml shake flasks containing 400 ml medium. The product was then harvested after 7 days of culture and clarified by centrifugation and filtration. Harvested media was further purified with CH1-XL resin using the manufacturer's protocol. The purified protein was buffer exchanged to PBS pH 7.0 containing 300 mM sucrose. The final product was characterized with respect to identity and purity.

### Characterization of the targeting module

Thermal stability of the targeting modules was assessed by nano-format of differential scanning fluorimetry (nanoDSF), backreflection, dynamic light scattering (DLS), static light scattering (SLS), size exclusion high performance liquid chromatography (SE-HPLC) and SDS-PAGE. Binding properties were assessed by surface plasmon resonance and/or cellular binding assay. Potency of the targeting modules was investigated by in vitro killing assays with target cells expressing CD19 and/or CD20.

**Figures 2** to **4** show the cellular binding potential assessed using the CD19 and/or CD20 positive target cell lines Oci-AML3 and NALM-6. Different concentrations of targeting modules according to the invention were incubated with cells, washed and quantified. A dose-response curve was obtained when the geometric mean fluorescence (MFI) was plotted against the TM concentration.

The data for the cellular binding of CD19 and CD20-binding TMs on the CD19 and/or CD20 positive target cell lines Oci-AML3 and NALM-6 were fitted using a four-parameter model with a variable slope for sigmoidal curves. The 50% effective concentration (EC₅₀) obtained from this model can be interpreted as a representative value of the TM affinity for the cells overexpressing the target receptor (see **Tab. 2**).

**Tab. 2 Targeting module affinity the cells overexpressing the target receptor. concentration of half-maximal binding (EC₅₀) determined by cellular binding assays.**

| **Targeting module** | **CD19 binding EC₅₀ [nM]** | **CD20 binding EC₅₀ [nM]** | **CD19/CD20 binding EC₅₀ [nM]** |
|---|---|---|---|
| R-TM 19/20_02 | 18.4 | 43.6 | 18.5 |
| R-TM 19/20_03 | 19.0 | 29.2 | not determined |
| R-TM 19/20_05 | 14.7 | 24.1 | 16.4 |
| R-TM 19/20_08 | 14.8 | 37.0 | 18.7 |
| R-TM19/20_11 | 5.2 | 62.0 | 24.3 |
| R-TM 19/20 15 | 3.7 | 45.5 | 26.6 |
| R-TM19/20_21 | 2.2 | 53.8 | 20.6 |
| R-TM19/20_23 | 3.4 | 23.0 | 14.2 |

### RevCAR T cells

For the genetical engineering to express RevCARs, a polynucleotide vector encoding the RevCAR and all necessary elements to ensure its expression in the genetically engineered immune cell is transferred into the immune cell. In particular, the RevCAR comprises a human signal peptide, tag, ECD (extracellular domain), TMD (transmembrane domain), ICD (intracellular domain).

The transfer of the vector can be performed by electroporation or transfection of nucleic acids or the help of viral vector systems like adeno-, adeno-associated, retro-, foamy- or lentiviral viral gene transfer.

The lentiviral gene transfer is applied for stable expression of RevCARs in immune cells by first constructing a lentiviral vector encoding for a selected RevCAR. The lentiviral vector is pLVX-EF1alpha UniCAR 28/ζ (Clontech, Takara Bio Group), in which the lentiviral parts of the vector are derived from the human immunodeficiency virus (HIV) and the MSC/IRES/ZxGreenl portion was replaced by the RevCAR construct.

The lentiviral particles are produced by transient transfection of human embryonal kidney (HEK) 293T (ACC 635) cells with the RevCAR encoding lentiviral vector plasmid and co-transfection with a group specific antigen (gag) and Polymerase (pol) encoding plasmid (psPAX2) plus a plasmid encoding for an envelope (pMD2.G). After transfection, the packaging plasmid expresses Gag and Pol protein of HIV-1. The plasmid MD2.G encodes the glycoprotein of the vesicular stomatitis virus (VSV-G). VSV-G protein is used to lentiviral vectors to transduce a broad range of mammalian cells. Various envelopes from different virus species can be utilized for this purpose. Lentiviral vectors can successfully pseudo type with the envelope glycoproteins (Env) of amphotropic murine leukemia virus (MLV) or the G protein of vesicular stomatitis virus (VSV-G), a modified envelope of the prototypic foamy virus (PFV) or chimeric envelope glycoprotein variants derived from gibbon ape leukemia virus (GaLV) and MLV.

Supernatants from transfected HEK293T cells are harvested 24 h to 96 h after transfection and virus particles are concentrated from the supernatant by ultracentrifugation or other methods. For lentiviral transduction of immune cells, peripheral blood mononuclear cells (PBMC) or isolated T cells are activated with mab specific for the CD3 complex, e.g. clone OKT3 or UCHT1, either given in solution or coated to plastic cell culture dishes or magnetic beads or a biodegradable polymer matrix. Activation of PBMC or isolated T cells is further enhanced by stimulating costimulatory pathways with mabs or ligands specific for CD27, CD28, CD134 or CD137 either alone or in combinations coated to plastic cell culture dishes or magnetic beads or a biodegradable polymer matrix and the supply with exogenous recombinant cytokines like interleukin (IL)-2, IL-7, IL-12, IL-15 and IL-21. Concentrated or non-concentrated virus particles are added to PBMC or T cell cultures 24 h to 96 h after initial administration of activating CD3 specific antibodies and/or antibodies specific for costimulatory receptors CD27, CD28, CD134 or CD137 and/or recombinant cytokines as single or multiple doses. T cell electroporation, transduction and expansion may be performed in open cell culture systems by manual handling or in closed partially or fully automated systems.

Stable transduction of T cells may be determined by flow cytometry after staining with tagcontaining molecules for surface expression of RevCARs or mabs directed against a fourth domain of RevCARs from day 3 onwards after the final administration of virus supernatant.

RevCAR transduced T cells can be propagated *in vitro* by culturing them under the supply of recombinant cytokines and activating anti-CD3 mabs.

In case the RevCAR harbors a tag the optional fourth domain, a peptide sequence forming a linear epitope for a mab, immune cells genetically modified to express RevCARs can be specifically propagated *in vitro* by coating a mab or antibody fragments thereof binding to the tag or the fourth RevCAR domain to the surface of culture dishes or to beads of any kind or a biodegradable polymer matrix, which are added to the cell culture at a defined ratio. The binding of surface-coated mabs to the RevCAR peptide domain induces cross-linkage of cell-surface expressed RevCARs and formation of an immune synapse, which leads to the activation of signal pathways specifically triggered by the signal domain of the RevCAR. Depending on the signal pathways induced, this may lead to enhance proliferation and sustained resistance against activation-induced cell death of the RevCAR-carrying immune cells and therefore enrichment of RevCAR genetically modified immune cells in a mixed population.

The tag or the optional fourth domain, a peptide sequence forming a linear epitope for a mab, can be further utilized to enrich and purify RevCAR-expressing immune cells from mixed populations. Enrichment and purification are performed with the help of a mab or antibody fragment thereof binding to the fourth RevCAR domain to either mark RevCAR-expressing cells for cell sorting or to transiently link the RevCAR expressing immune cell to small particles, which can be utilized for cell isolation. In one aspect, RevCAR-engrafted immune cells are incubated with the mab recognizing the fourth domain. Next, magnetic beads are added, which are conjugated with antibodies or fragments thereof directed against the species- and isotype-specific heavy and light chains of the mab binding to the optional fourth domain. Thus, RevCAR-expressing immune cells and magnetic beads are linked and are trapped and separated from other immune cells in a magnetic field.

### Cytotoxicity assay

The potency of CD19 and CD20-binding TMs to induce a tumor cell elimination by RevCAR-T cells was tested using a suspension cell-based co-cultivation assay with the acute myeloid leukemia cell line Oci-AML3 (**Fig. 5** and **6**) and acute lymphoblastic leukemia cell line NALM-6 (**Fig. 7**) in the presence of variable concentrations of the targeting module. Switchable CAR-T cells were incubated with the target cells at a E:T ratio of 1:2 in the presence of various TM concentrations for 48 h. As CD19- and/or CD20-positive target cells the human cell line Oci-AML3 (**Fig. 5** and **6**) or NALM-6 (**Fig. 7**), respectively, was used which was stained with efluor prior setup. Target cells were quantified by flow cytometry and lysis was calculated normalizing the cell count of each sample to a control sample where only tumor cells were plated. Data were fitted with a four-parameter model with a variable slope for sigmoidal curves. The calculated EC₅₀ value can be interpreted as a representative value for the TM potency against these tumor cells.

**Tab. 3 Cell lysis: concentration of half-maximal killing (EC₅₀).**

| **Targeting module** | **CD19 killing EC₅₀ [pM]** | **CD20 killing EC₅₀ [pM]** | **CD19/CD20 killing EC₅₀ [pM]** |
|---|---|---|---|
| R-TM 19/20_02 | 3.8 | 7.2 | 2.4 |
| R-TM 19/20_03 | 7.9 | 4.3 | not determined |
| R-TM 19/20_05 | 6.2 | 9.8 | 3.1 |
| R-TM 19/20_08 | 7.1 | 14.2 | 3.8 |
| R-TM19/20_11 | 3.8 | 17.9 | 2.5 |
| R-TM19/20_15 | 1.2 | 10.7 | 1.5 |
| R-TM19/20_21 | 1.4 | 12.6 | 0.5 |
| R-TM19/20_23 | 1.0 | 2.1 | 0.5 |

### Cited non-patent literature

Brudno JN, Kochenderfer JN (2016) Toxicities of chimeric antigen receptor T cells: recognition and management. Blood 127, 3321-3330.
Cartellieri M, Bachmann M, Feldmann A, Bippes C, Stamova S, Wehner R, Temme A, Schmitz M (2010) Chimeric Antigen Receptor-Engineered T Cells for Immunotherapy of Cancer J. Biomed. Biotechnol. Article ID 956304, doi: 10.1155/2010/956304.
Cartellieri M, Feldmann A, Koristka S, Arndt C, Loff S, Ehninger A, von Bonin M, Bejestani EP, Ehninger G, Bachmann MP (2016) Switching CAR T cells on and off: a novel modular platform for retargeting of T cells to AML blasts. Blood cancer J. 6 (8), e458.
Darowski D, Kobold S, Jost C, Klein C (2019) Combining the best of two worlds: highly flexible chimeric antigen receptor adaptor molecules (CAR-adaptors) for the recruitment of chimeric antigen receptor T cells. MAbs. 11 (4), 621-631.
Fedorov VD, Themeli M, Sadelain M (2013) PD-1- and CTLA-4-based inhibitory chimeric antigen receptors (iCARs) divert off-target immunotherapy responses. Sci. Transl. Med. 5 (215), 215ra172.
Feldmann A, Hoffmann A, Bergmann R, Koristka S, Berndt N, Arndt C, Rodrigues Loureiro L, Kittel-Boselli E, Mitwasi N, Kegler A, Lamprecht C, González Soto KE, Bachmann M (2020) Versatile chimeric antigen receptor platform for controllable and combinatorial T cell therapy. Oncoimmunology. 9 (1), 1785608.
Finney HM, Akbar AN, Lawson AD (2004) Activation of resting human primary T cells with chimeric receptors: costimulation from CD28, inducible costimulator, CD134, and CD137 in series with signals from the TCR zeta chain. J. Immunol. 172 (1), 104-113.
Frigault MJ, Lee J, Basil MC, Carpenito C, Motohashi S, Scholler J, Kawalekar OU, Guedan S, McGettigan SE, Posey AD Jr, Ang S, Cooper LJ, Platt JM, Johnson FB, Paulos CM, Zhao Y, Kalos M, Milone MC, June CH (2015) Identification of chimeric antigen receptors that mediate constitutive or inducible proliferation of T cells. Cancer Immunol. Res. 3 (4), 356-367.
Gong MC, Latouche JB, Krause A, Heston WDW, Bander NH, Sadelain M (1999) Cancer patient T cells genetically targeted to prostate-specific membrane antigen specifically lyse prostate cancer cells and release cytokines in response to prostate-specific membrane antigen. Neoplasia. 1 (2), 123-127.
Guedan S, Posey AD, Shaw C, Wing A, Da T, Patel PR, McGettigan SE, Casado-Medrano V, Kawalekar OU, Uribe-Herranz M, Song D, Melenhorst JJ, Lacey SF, Scholler J, Keith B, Young RM, June CH (2018) Enhancing CAR T cell persistence through ICOS and 4-1BB costimulation. JCI Insight. 3 (1), 96976.
Guedan S, Madar A, Casado-Medrano V, Shaw CE, Wing A, Liu F, Young RM, June CH, Posey AD (2020) Single residue in CD28-costimulated CAR T cells limits long-term persistence and antitumor durability. J Clin Invest. 133215.
Hombach A, Sent D, Schneider C, Heuser C, Koch D, Pohl C, Seliger B, Abken H (2001) T-cell activation by recombinant receptors: CD28 costimulation is required for interleukin 2 secretion and receptor-mediated T-cell proliferation but does not affect receptor-mediated target cell lysis. Cancer Res. 61 (5), 1976-1982.
Kagoya Y, Tanaka S, Guo T, Anczurowski M, Wang CH, Saso K, Butler MO, Minden MD, Hirano N (2018) A novel chimeric antigen receptor containing a JAK-STAT signaling domain mediates superior antitumor effects. Nat Med. 24 (3), 352-359.
Kittel-Boselli E, Soto KEG, Loureiro LR, Hoffmann A, Bergmann R, Arndt C, Koristka S, Mitwasi N, Kegler A, Bartsch T, Berndt N, Altmann H, Fasslrinner F, Bornhäuser M, Bachmann MP, Feldmann A (2021) Targeting Acute Myeloid Leukemia Using the RevCAR Platform: A Programmable, Switchable and Combinatorial Strategy. Cancers (Basel). 13(19), 4785.
Lamers CH, Sleijfer S, Willemsen RA, Debets R, Kruit WHJ, Gratama JW, Stoter G (2004) Adoptive immuno-gene therapy of cancer with single chain antibody [scFv(lg)] gene modified T lymphocytes. J. Biol. Regul. Homeost. Agents. 18 (2), 134-140.
Milone MC, Fish JD, Carpenito C, Carroll RG, Binder GK, Teachey D, Samanta M, Lakhal M, Gloss B, Danet-Desnoyers G, Campana D, Riley JL, Grupp SA, June CH (2009) Chimeric receptors containing CD137 signal transduction domains mediate enhanced survival of T cells and increased antileukemic efficacy in vivo. Mol. Ther. 17 (8), 1453-1464.
Morgan RA, Yang JC, Kitano M, Dudley ME, Laurencot CM, Rosenberg SA (2010) Case Report of a Serious Adverse Event Following the Administration of T Cells Transduced With a Chimeric Antigen Receptor Recognizing ERBB2. Mol. Ther. 18, 843-851.
Pinthus JH, Waks T, Kaufman-Francis K, Schindler DG, Harmelin A, Kanety H, Ramon J, Eshhar Z (2003) Immuno-gene therapy of established prostate tumors using chimeric receptor-redirected human lymphocytes. Cancer Res. 63 (10), 2470-2476.
Reinke AW, Grant RA, Keating AE (2010) A Synthetic Coiled-Coil Interactome Provides Heterospecific Modules for Molecular Engineering. JACS 132, 6025-6031.
Sotillo E, Barrett DM, Black K., Bagashev A, Oldridge D, Wu G, Sussman R, Lanauze C, Ruella M, Gazzara MR, Martinez NM, Harrington CT, Chung EY, Perazzelli J, Hofmann TJ, Maude SL, Raman P, Barrera A, Gill S, Lacey SF, Melenhorst JJ, Allman D, Jacoby E, Fry T, Mackall C, Barash Y, Lynch KW, Maris JM, Grupp SA, Thomas-Tikhonenko A (2015) Convergence of Acquired Mutations and Alternative Splicing of CD19 Enables Resistance to CART-19 Immunotherapy. Cancer Discov. 5, 1282-1295.
Titov A, Petukhov A, Staliarova A, Motorin D, Bulatov E, Shuvalov O, Soond SM, Piacentini M, Melino G, Zaritskey A, Barlev NA (2018) The biological basis and clinical symptoms of CAR-T therapy-associated toxicites. Cell Death Dis 9, 897.
Töpfer K, Cartellieri M, Michen S, Wiedemuth R, Müller N, Lindemann D, Bachmann M, Füssel M, Schackert G, Temme A (2015) DAP12-based activating chimeric antigen receptor for NK cell tumor immunotherapy. J. Immunol. 194 (7), 3201-3212.
Zhang T, Lemoi BA, Sentman CL (2005) Chimeric NK-receptor-bearing T cells mediate antitumor immunotherapy. Blood. 106 (5), 1544-1551.

### List of reference signs

- 1: targeting module
- 2: switchable chimeric antigen receptor
- 3: effector cell
- 4: target cell

## Claims

1. A targeting module comprising
i) at least one CD19-binding domain comprising one of the sequences selected from SEQ ID No. 1 to SEQ ID No. 3 or a sequence identity of at least 95 % with one of the sequences selected from SEQ ID No. 1 to SEQ ID No. 3,
ii) at least one CD20-binding domain comprising SEQ ID No. 4, and
iii) a tag-binding domain or a tag.

2. The targeting module according to claim 1, wherein the tag is a peptide epitope tag.

3. The targeting module according to claim 2, wherein the peptide epitope tag is a myc-tag, a His-tag, a short linear peptide sequence from yeast transcription factor GCN4, a leucine zipper sequence or a short linear peptide sequence from a human nuclear protein, preferably from the human La protein.

4. The targeting module according to one of the claims 1 to 3, wherein at least one CD20-binding domain comprises one of the sequences selected from SEQ ID No. 5 to SEQ ID No. 7 or with a sequence identity of at least 95 % with one of the sequences selected from SEQ ID No. 5 to SEQ ID No. 7.

5. The targeting module according to one of the claims 1 to 4, wherein the length is in the range of 200 to 1600 amino acids.

6. The targeting module according to one of the claims 1 to 5 comprising at least three chains comprising
- one amino acid sequence selected from the group comprising SEQ ID No. 39, SEQ ID No. 46, SEQ ID No. 47, SEQ ID No. 72, SEQ ID No. 73, SEQ ID No. 74, SEQ ID No. 75, and SEQ ID No. 76, and
- one amino acid sequence selected from the group comprising SEQ ID No. 44 and SEQ ID No. 45, and
- one amino acid sequence selected from the group comprising SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, and SEQ ID No. 71.

7. A targeting module according to one of the claims 1 to 6 for use in the treatment of cancer, infectious disease or autoimmune disease,
wherein the targeting module is administered in combination with a cell comprising a nucleotide sequence encoding a switchable chimeric antigen receptor,
wherein the switchable chimeric antigen receptor comprises
- a tag-binding domain or a tag,
- an extracellular hinge and a transmembrane domain and
- a signal transduction domain,
wherein the tag-binding domain of the targeting module binds to the tag of the switchable chimeric antigen receptor or the tag of the targeting module binds to the tag-binding domain of the switchable chimeric antigen receptor.

8. The targeting module for use in the treatment of cancer, infectious disease or autoimmune disease according to claim 7,
wherein the targeting module is administered in combination with a cell comprising a nucleotide sequence encoding a switchable chimeric antigen receptor and at least one further targeting module,
wherein the at least one further targeting module comprises at least one target cell-binding domain and a tag-binding domain or a tag,
wherein the at least one target cell-binding domain is an antibody, antibody fragment, a protein, a peptide or a low molecular weight organic ligand that binds to a surface antigen selected from the group comprising CD2, CD3, CD4, CD5, CD7, CD8, CD10, CD15, CD22, CD23, CD25, CD30, CD33, CD38, CD44, CD44v6, CD52, CD66a, CD66b, CD66c, CD66d, CD66e, CD66f, CD90, CD99, CD123, CD133, CD135, CD150, CD181, CD182, CD184, CD223, CD229, CD269, CD273, CD274, CD276, CD279, CD319, CD366, CD371, cytokine receptors, CXCR4, c-Met, mesothelin, a member of the epidermal growth factor receptor family or a mutant thereof, a member of the tumor necrosis factor receptor superfamily, a claudin, an ephrin, an ephrin receptor, a fucosyl transferase, a prostate specific antigen, an embryonic antigen, a member of the vascular endothelia growth factor family, epithelial cell adhesion molecule, alpha-fetoprotein, a member of the intercellular adhesion molecule family, a C-type lectin, an integrin, a member of the mucin protein family, a follicle-stimulating hormone receptor, a high molecular weight-melanoma associated antigen, a folate binding protein, a folate receptor, a somatostatin receptor, a ligand of the NKG2D receptor, a member of the epithelia glycoprotein family, a diasialoganglioside, a glypican, a G protein-coupled receptor, a human papillomavirus protein, cancer/testis antigen, fibroblast activation protein, a member of the carbonic anhydrase family, a member of the carbohydrate antigen family, a Notch ligand, melanoma-associated chondroitin sulfate proteoglycan, glycoprotein A33, guanylate cyclase 2C and tumor-specific glycan,
wherein the targeting module and the at least one further targeting module comprise different target cell-binding domains, and identical tag-binding domains or tags.

9. A pharmaceutical composition comprising the targeting module according to one of the claims 1 to 6 and a pharmaceutically acceptable thinner or carrier.

10. The pharmaceutical composition according to claim 9 comprising at least one further targeting module,
wherein the at least one further targeting module comprises at least one target cell-binding domain and a tag-binding domain or a tag,
wherein the at least one target cell-binding domain is an antibody, antibody fragment, a protein, a peptide or a low molecular weight organic ligand that binds to surface antigens selected from the group comprising CD2, CD3, CD4, CD5, CD7, CD8, CD10, CD15, CD22, CD23, CD25, CD30, CD33, CD38, CD44, CD44v6, CD52, CD66a, CD66b, CD66c, CD66d, CD66e, CD66f, CD90, CD99, CD123, CD133, CD135, CD150, CD181, CD182, CD184, CD223, CD229, CD269, CD273, CD274, CD276, CD279, CD319, CD366, CD371, cytokine receptors, CXCR4, c-Met, mesothelin, a member of the epidermal growth factor receptor family or a mutant thereof, a member of the tumor necrosis factor receptor superfamily, a claudin, an ephrin, an ephrin receptor, a fucosyl transferase, a prostate specific antigen, an embryonic antigen, a member of the vascular endothelia growth factor family, epithelial cell adhesion molecule, alpha-fetoprotein, a member of the intercellular adhesion molecule family, a C-type lectin, an integrin, a member of the mucin protein family, a follicle-stimulating hormone receptor, a high molecular weight-melanoma associated antigen, a folate binding protein, a folate receptor, a somatostatin receptor, a ligand of the NKG2D receptor, a member of the epithelia glycoprotein family, a diasialoganglioside, a glypican, a G protein-coupled receptor, a human papillomavirus protein, cancer/testis antigen, fibroblast activation protein, a member of the carbonic anhydrase family, a member of the carbohydrate antigen family, a Notch ligand, melanoma-associated chondroitin sulfate proteoglycan, glycoprotein A33, guanylate cyclase 2C and tumor-specific glycan,
wherein the targeting module and the at least one further targeting module comprise different target cell-binding domains, and identical tag-binding domains or tags.

11. A kit comprising
a) a targeting module comprising
i) at least one CD19-binding domain comprising one of the sequences selected from SEQ ID No. 1 to SEQ ID No. 3 or a sequence identity of at least 95 % with one of the sequences selected from SEQ ID No. 1 to SEQ ID No. 3,
ii) at least one CD20-binding domain comprising SEQ ID No. 4, and
iii) a tag-binding domain or a tag, and
b) a vector or a cell comprising a nucleotide sequence encoding a switchable chimeric antigen receptor,
wherein the switchable chimeric antigen receptor comprises
- a tag-binding domain or tag,
- an extracellular hinge and a transmembrane domain and
- a signal transduction domain,
wherein the tag-binding domain of the targeting module binds to the tag of the switchable chimeric antigen receptor or the tag of the targeting module binds to the tag-binding domain of the switchable chimeric antigen receptor.

12. The kit according to claim 11, wherein the tag is a myc-tag, a His-tag, a short linear peptide sequence from yeast transcription factor GCN4, a leucine zipper sequence or a short linear peptide sequence from a human nuclear protein, preferably from the human La protein.

13. The kit according to claim 11 or 12, wherein the tag-binding domain is an antibody or an antibody fragment binding to a myc-tag, a His-tag, a short linear peptide sequence from yeast transcription factor GCN4, a leucine zipper sequence or a short linear peptide sequence from a human nuclear protein, preferably from a human La protein.

14. The kit according to one of the claims 11 to 13, wherein the at least one CD20-binding domain comprises one of the sequences selected from SEQ ID No. 5 to SEQ ID No. 7 or with a sequence identity of at least 95 % with one of the sequences selected from SEQ ID No. 5 to SEQ ID No. 7.

15. The kit according to one of the claims 11 to 14, wherein the length of the targeting module is in the range of 200 to 1600 amino acids.

16. The kit according to one of the claims 11 to 15, wherein the targeting module comprises at least three chains comprising
- one amino acid sequence selected from the group comprising SEQ ID No. 39, SEQ ID No. 46, SEQ ID No. 47, SEQ ID No. 72, SEQ ID No. 73, SEQ ID No. 74, SEQ ID No. 75, and SEQ ID No. 76, and
- one amino acid sequence selected from the group comprising SEQ ID No. 44 and SEQ ID No. 45, and
- one amino acid sequence selected from the group comprising SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, and SEQ ID No. 71.

17. The kit according to one of the claims 11 to 16, wherein the extracellular hinge and a transmembrane domain is selected from the group comprising a hinge and transmembrane domain of human CD8α, CD28, ICOS (CD278), parts of a NK cell receptor, parts of the constant region of an antibody or mutants and combinations thereof.

18. The kit according to one of the claims 11 to 17, wherein the signal transduction domain comprises at least two signal transduction domains independently selected from a cytoplasmic region of a CD3 chain, CD28, CD137 (4-1BB), CD134 (OX40), CD278 (ICOS), DAP10, CD27, programmed cell death-1 (PD-1), cytotoxic T-lymphocyte antigen 4 (CTLA-4), DAP12, CD122 (interleukin-2 receptor β), CD132 (interleukin-2 receptor γ), CD127 (interleukin-7 receptor α), CD360 (interleukin-21 receptor), an activating Fc receptor and mutants thereof.

19. The kit according to one of the claims 11 to 18 further comprising at least one further targeting module,
wherein the at least one further targeting module comprises at least one target cell-binding domain and a tag-binding domain or a tag,
wherein the at least one target cell-binding domain is an antibody, antibody fragment, a protein, a peptide or a low molecular weight organic ligand that binds to surface antigens selected from the group comprising CD2, CD3, CD4, CD5, CD7, CD8, CD10, CD15, CD22, CD23, CD25, CD30, CD33, CD38, CD44, CD44v6, CD52, CD66a, CD66b, CD66c, CD66d, CD66e, CD66f, CD90, CD99, CD123, CD133, CD135, CD150, CD181, CD182, CD184, CD223, CD229, CD269, CD273, CD274, CD276, CD279, CD319, CD366, CD371, cytokine receptors, CXCR4, c-Met, mesothelin, a member of the epidermal growth factor receptor family or a mutant thereof, a member of the tumor necrosis factor receptor superfamily, a claudin, ephrin, an ephrin receptor, a fucosyl transferase, a prostate specific antigen, an embryonic antigen, a member of the vascular endothelia growth factor family, epithelial cell adhesion molecule, alpha-fetoprotein, a member of the intercellular adhesion molecule family, a C-type lectin, an integrin, a member of the mucin protein family, a follicle-stimulating hormone receptor, a high molecular weight-melanoma associated antigen, a folate binding protein, a folate receptor, a somatostatin receptor, a ligand of the NKG2Dreceptor, a member of the epithelia glycoprotein family, a diasialoganglioside, a glypican, a G protein-coupled receptor, a human papillomavirus protein, cancer/testis antigen, fibroblast activation protein, member of the carbonic anhydrase family, member of the carbohydrate antigen family, Notch ligand, melanoma-associated chondroitin sulfate proteoglycan, glycoprotein A33, guanylate cyclase 2C and tumor-specific glycan,
wherein the targeting module and the at least one further targeting module comprise different target cell-binding domains, and identical tag-binding domains or tags.

20. The kit according to one of the claims 11 to 19, wherein the targeting module and/or the cell comprising a nucleotide sequence encoding a switchable chimeric antigen receptor are in the form of a pharmaceutical composition.

21. The kit according to one of the claims 11 to 20 for use in the treatment of cancer, infectious disease or autoimmune disease.
